# EUROPEAN PATENT APPLICATION

(11) **EP 3 892 307 A1**
(43) Date of publication of application: **13.10.2021**
(21) Application number: 20382392.7
(22) Date of filing: 12.05.2020
(51) Int. Cl.: A61L 2/20, A61L 2/24

(54) **SYSTEMS AND METHODS FOR DISINFECTING ITEMS WITH OZONE GAS**

(30) Priority: 07.04.2020 EP 20382283
(71) Applicant: Jeanología, S.L., 46980 Paterna, Valencia (ES)
(72) Inventor: SILLA VIDAL, Enrique, 46980 Paterna (Valencia) (ES); ALBERT REVERT, Vicente, 46980 Paterna (Valencia) (ES); PUCHOL ESTORS, Victoria, 46980 Paterna (Valencia) (ES)
(74) Representative: González Poveda, Sara

(57) **Abstract**

A device (system) and methods for disinfecting items with ozone gas. The device (system) comprises a disinfection chamber that has a door via which the items are being placed, an ozone generator, an ozone destroy system and a control unit. The device (system) optionally comprises a heating system for increasing the temperature in the disinfection chamber, and/or comprises a UV radiation source. The items to be disinfected are preferably wearables, such as clothes, personal protective equipment, helmets, goggles, face masks.

## Description

### Technical Field

The present invention refers to the technical field of disinfecting (sanitizing) items, such as wearables, using ozone gas. Disinfecting items can also be sanitizing items. More specifically the present application describes devices, vehicles and methods for disinfecting wearables using ozone gas. In the context of the present application a device described herein may be also considered as being an apparatus or a system or a machine. A wearable should be understood as being any type of item that may be worn on the human body, such as for example an article of clothing, shoes, a clothing accessory, a personal protective equipment item, a sports wearable o handheld item and the like. Alternatively, the devices, vehicles and methods described herein may be used for disinfecting any item that fits within the devices or the vehicles. It is of particular interest using the present invention for disinfecting the wearables from pathogens such as biological pathogens, bacteria, germs and viruses, for example coronavirus, which may be present on the wearables as a result of the wearable's location and/or use, such as for example the wearable's presence and use in a hospital with infected patients or workers, or within an apparel shop wherein some of the workers or clients carrying a pathogen may contaminate said wearables, or more generally by the proximity of the wearable to a patient or source that releases said pathogen. Said wearables upon efficiently disinfected may be re-used, and this has tremendous health and economic advantages for their users and for the society. Disinfecting refers to fully or partially removing or killing or eliminating or neutralizing said pathogens from said items or wearables.

### Prior art.

The 2019-20 coronavirus pandemic which has been caused by the ongoing spread of the SARS-COV-2 virus that causes to humans the COVID-19 disease, has a devastating impact on the public health and the economy at a global scale. Efforts to fight this pandemic as well as other epidemics include the adaptation and application of practices related to disinfecting everyday use items and surfaces. It is well known that a human carrier of biological pathogens, such as viruses or bacteria, may pass said pathogens to a surface, and often said pathogens may stay on said surface for several hours or days without losing their ability to infect others who will come into contact with the surface and obtain therefrom the pathogens. This is often the case with the surfaces of wearables such as for example the surfaces of personal protective equipment such as googles, scrubs and protection masks, used by doctors, nurses and patients in hospitals having patients being infected with pathogens. It is highly desirable disinfecting said surfaces, items and wearables as often as possible, and preferably every time before they are being used or worn. By disinfecting them, meaning eliminating or removing or neutralizing any of the pathogens on them, said items or wearables are safe to use or re-use.

There exist several known methods for killing biological pathogens. One of them concerns the use of ozone gas. It is well known that ozone gas can kill bacteria and viruses. For this reason, ozone gas is often used for disinfecting water, such as wastewater. In fact, often, disinfecting wastewater with ozone is a preferred technique over others because comparatively the former is considered to be eco-friendlier and more cost effective.

In view of said proven use of ozone gas to disinfect and eliminate biological pathogens, and in view of the proven fact that epidemics such as the 2019-20 pandemic have caused a great need for the widespread adaptation of techniques for disinfecting everyday items such as wearables for the everyday use and re-use of said items, the problem is how to best implement ozone gas for disinfecting and thusly safely reusing everyday items such as wearables and personal protective equipment, especially when the demand for safely using and reusing said items is temporarily increased and/or spiking.

### Description of the invention

It is a first object of the invention to provide a device for disinfecting items, particularly wearables. The device may also be considered as being a machine or a system or an apparatus.

It is a second object of the intention to provide a system for disinfecting items, particularly wearables, wherein said system comprises a vehicle that comprises the device related to the first object of the invention. Said device is located on or within said vehicle. Hence the system related to the second object of the invention, may also be considered as being a vehicle. The vehicle preferably is a truck and preferably has a trunk. Preferably, the device is located on/within said trunk i.e. the device has been loaded on/within the trunk. A non-limiting example of a truck is a pickup truck or a station wagon.

It is a further object of the invention to provide methods for disinfecting items, particularly wearables, using the device related to the first object and/or the system related to the second object.

The efficient use of ozone gas for disinfecting wearables and/or other everyday items requires having machines, meaning devices or systems or apparatuses, that are easy and safe to use, they are mobile or can be moved easily between places, and have a proper form so they can be used in establishments wherein there is a high demand for disinfecting items. Non limiting examples of such establishments are healthcare establishments, stores such as apparel and clothing stores, factories or other commercial buildings and installations, places wherein food is produced or processed, public service buildings and the like. For example, in a healthcare establishment such as in a hospital it is required to use ozone gas for disinfecting personal protection equipment such as face masks, scrubs and googles for safely reusing said equipment as often as possible and/or as often as is required depending on the viral load in said hospitals at any given time. Likewise, in a clothing store and especially in times of a pandemic, it is desirable that the cloths therein are disinfected every time before or after they are used or worn or tried by the clients.

Therefore, a technical problem is how to design machines, systems, devices for frequently or daily disinfecting items for reusing said items, and especially for doing said disinfection close, or at the place wherein, said items are used. This technical problem entails the additional problems of how to make these machines to be safe, easy to use, cost-effective, compact, mobile or easily movable between places, energy efficient, and easy to produce at a mass scale and at a comparatively low cost. The present invention addresses all the aforementioned problems.

The invention in its first aspect concerns a device (i.e. an apparatus or system) for disinfecting items, such as wearables, the device comprising:
- a disinfection chamber which has a door and is configured for receiving within it, meaning to its interior, the items to be disinfected;
- an ozone generator connected to the disinfection chamber and configured to generate ozone gas and supply the latter to within (the interior of) the disinfection chamber when the system is operated;
- an ozone destroy system connected to disinfection chamber and configured to receive from within (the interior of) the latter ozone gas and to convert said ozone gas to a non-toxic gaseous product, and to preferably release said non-toxic gaseous product;
- sensing means configured for sensing at least one operational parameter and generating and transmit a corresponding sensing signal;
- a control unit that comprises a power supply for powering the device, a computer and user command means configured for receiving operation commands from a user, the computer preferably being a programmable logic controller, and the control unit is configured to control the operation of the device according to the operation commands, and is configured to receive the sensing signal and inhibit the operation of the system when the sensing signal does not correspond to a safety condition.

The device may also be considered as being a machine or a system or an apparatus. The ozone destroy means ensure that the exhaust product of the device is non-toxic and poses no harm to the users. The sensing means, which in a non-limiting preferable example comprise a first ozone gas sensor installed within the disinfection chamber and configured to measure the ozone concentration in gas, and a second ozone gas sensor installed outside the chamber and configured to sense whether any undesired leakage of ozone takes place, and a sensor that detects whether the door is closed or open, solves the technical problem on how to know whether the operational parameters are within any specifications dictated by the user or the device manufacturer so that the disinfection is efficient and safe. The control unit and in particular the computer or its equivalent programmable logic controller (PLC) provide for an accurate and well controlled operation of the device, and preferably coordinate the operation of the electronic or electrical or electromechanical components therein when the system is used/operated. The user control means, which in an example comprises a touch screen connected to the PLC, allows for an easy and/or advanced use of the system by the users. In a non-limiting example, the safety condition is that the door is closed.

Preferably the ozone generator is configured to generate ozone gas at a rate of between 1 grams of ozone per hour to 100 grams of ozone per hour, and preferably of between 1 grams of ozone per hour to 60 grams of ozone per hour; at lower rates the ozone gas concentration may not be sufficient for achieving a sufficient degree of disinfection, and at higher rates the ozone gas concentration may be too high causing deterioration of the items to be disinfected.

More preferably, the invention in its first aspect concerns a device (system or apparatus) for disinfecting (sanitizing) wearables, the device comprising:
- a main body that has a height, a length and a width, and comprises a disinfection chamber which has a door and is configured for receiving within it and via the door the wearables to be disinfected;
- a tubing system that has an ozone inlet, an ozone outlet, an exhaust inlet, an exhaust outlet and an end exhaust, wherein the tubing system is connected to the disinfection chamber via the ozone outlet and the exhaust inlet, and is configured to, during an operation of the device by a user, supply to the disinfection chamber ozone gas via the ozone outlet, and to receive from the disinfection chamber ozone gas via the exhaust inlet;
- an ozone generator connected to the tubing system and configured to generate ozone gas and supply via the ozone inlet said ozone gas to the tubing system;
- an ozone destroy system connected to the tubing system and is configured to receive from the latter via the exhaust outlet ozone gas that has been received by the tubing system via the exhaust inlet, and to convert said ozone gas to a non-toxic gaseous product, and to supply said non-toxic gaseous product to the end exhaust;
- sensing means configured for sensing at least one operational parameter and generating and transmit a corresponding sensing signal;
- a control unit that comprises a power supply for powering the device, a computer and user command means configured for receiving operation commands from a user, the computer preferably being a programmable logic controller, and the control unit is configured to control the operation of the device according to the operation commands, and is configured to receive the sensing signal and inhibit the operation of the system when the sensing signal does not correspond to a safety condition.

As indicate further above, optionally and preferably the ozone generator is configured to for generating ozone gas at a rate of between 1 grams of ozone per hour to 100 grams of ozone per hour, and preferably of between 1 grams of ozone per hour to 60 grams of ozone per hour.

Optionally and preferably the device comprises a heating system that is configured to increase the temperature inside the disinfection chamber. When the ozone is supplied to the disinfection chamber while the gaseous atmosphere (gas environment) inside said chamber is hot, then the items inside said chamber are being sanitized better and faster It is contemplated by the inventors of the present invention that when the heating system causes the temperature inside the disinfection chamber to increase, the supplied ozone neutralizers faster and more efficient any pathogens on the items inside the disinfection chamber. For achieving better, easier and more accurate control and operation of the heating system, optionally and preferably the heating system is connected to and configured to be controllable (controlled) by the control unit, and the latter is configured to control the heating system.

Optionally and preferably the heating system comprises a heating element installed (located) inside the disinfection chamber or inside the tubing system that is connected to and communicates with said disinfection chamber. The heating element can for example be an electrical resistance or a heating (IR) lamp or a heat exchanger that produces heat when supplied with electrical current. Optionally and preferably the heating element comprises an electrical resistance that preferably is configured to operate at a nominal power in the range of 1 kW to 10 kW, and more preferably in the range of 1 kW to 3 kW. At said nominal powers, the temperature in the disinfection chamber can be increased quickly when the heating system is operated. Likewise, optionally and preferably the heating system comprises a temperature sensor that is configured to measure the temperature inside the disinfection chamber or inside the tubing system. Preferably the measurement by the temperature sensor is used as feedback for/when operating/activating/deactivating/powering-on/powering-off the heating element. In a non-limiting example, the temperature sensor is a Pt100 sensor.

Preferably the heating system heats the gaseous atmosphere inside the disinfection chamber. Said gaseous atmosphere is the mixture of any gases inside the disinfection chamber. Therefore, said gaseous atmosphere may be the air inside the disinfection chamber. Heating said gaseous atmosphere results to heating the items (wearables) inside the disinfection chamber. For heating said gaseous atmosphere, optionally and preferably the heating system comprises an auxiliary suction pump that is configured to force any gas from inside the disinfection chamber to flow by the heating element and to subsequently flow inside the disinfection chamber. The auxiliary suction pump may be technically similar to the suction pump that is described further below. Likewise, optionally and preferably, the tubing system comprises a tubing circuit, and the heating element and the auxiliary pump are installed (located, connected to) at said tubing circuit, and the tubing system and heating system, when the latter is operated, are configured to extract (any) gas from the disinfection chamber via said auxiliary suction pump, pass (move) said gas via the tubing circuit and (via or close by or by) the heating element thereat, heat said gas with the heating element, and reintroduce the thusly heated gas into the disinfection chamber. With the aforementioned optional configuration, the heating system can heat the gaseous atmosphere (any gas) of the disinfection chamber. Preferably the tubing circuit is separate from the rest of the tubing system so that it is avoided heating the ozone gas before the latter enters the disinfection chamber For this reason, optionally and preferably, the tubing circuit comprises a tubing circuit inlet connected (installed on) to the disinfection chamber, a tubing circuit outlet connected to (installed on) the disinfection chamber, and the auxiliary suction pump when operated is configured to extract gas from the disinfection chamber via said tubing circuit inlet, and reintroduce the gas into disinfection chamber via said tubing circuit outlet. Preferably, the temperature inside the disinfection chamber is the temperature of a gaseous atmosphere that is inside said disinfection chamber, or is the temperature of a gaseous atmosphere that is inside the tubing circuit connected to said disinfection chamber, or is the temperature of any part of said gaseous atmosphere, and further preferably is the temperature measured by the temperature sensor. It is contemplated that in the system (device, apparatus) the ozone gas sanitizes (disinfects) better the items (e.g. the wearables, clothes) when the temperature of the latter is approximately of between 50°C and 70°C. For this reason, the best desired disinfection temperature (desired sanitization temperature) of the gaseous atmosphere is of between 80°C and 100°C, because it must be considered that in practice during a sanitization/disinfection cycle is some cases there will be a difference between the temperature of the items and the temperature of the gaseous atmosphere surrounding said items. Therefore, optionally and preferably the heating system is configured to increase the temperature inside the disinfection chamber to a desired disinfection temperature, said desired disinfection temperature preferably being of between 25°C and 100°C, and more preferably of between 40°C to 90°C, and most preferably of between 60°C and 90°C.

According to the above, optionally and preferably in the device (system) of the first aspect of the invention the tubing system further comprises a tubing circuit that has a circuit inlet and a circuit outlet, said tubing circuit being connected to the disinfection chamber via the circuit inlet and the circuit outlet, and moreover the device further comprises a heating system that when operated is configured to increase a temperature inside the disinfection chamber, the heating system comprising a heating element, an auxiliary suction pump, and a temperature sensor, the heating element when operated being configured to heat inside the tubing circuit, the auxiliary suction pump when operated being configured to force any gas from inside the disinfection chamber to successively exit said disinfection chamber via said circuit inlet, flow inside said tubing circuit, and reenter the disinfection chamber via the circuit outlet.

Optionally and preferably, a method or an operated command related to operating the system (device), comprises operating or activating or deactivating the heating system and one or more components of the heating system, during any of the following and combinations thereof:
- before injecting (supplying) ozone to the disinfection chamber
- when injecting ozone to the disinfection chamber
- after injecting ozone to the disinfection chamber.

The ventilation system that is described further down, may contribute to homogenizing the temperature inside the disinfection chamber. For sanitizing some items with the device, for example when applying a sanitization cycle, the heating system heats for a total period of time that can also be called a heating duration.

Optionally and preferably, the device is shaped and sized as a standalone closet. Such a device can be used in a cloths or apparel or shoe store and the like, for disinfecting cloths or shoes or other wearables therein. For example, such device can be located at or within the changing rooms and be used by the shoppers or workers in the stores for disinfecting the wearables every time before and/or after said wearables are tried. Of course, such a device is appropriately shaped and sized so that preferably a user while standing can place or hang within it the wearables.

Optionally and preferably the device and/or the disinfection chamber and/or the aforementioned main body has the shape of a cuboid. In that case the device would be easy to fabricate, install and be fitted at the place of use.

Optionally, the device has the shape and size of a benchtop instrument; this is a particularly preferred option in the case that the device is intended to be used for disinfecting small wearables such as face masks or shoes, and/or when the machine is to be placed on work benches, such for example on a workbench in a hospital or in a laboratory wherein the workers may need to frequently disinfect small personal protective equipment items. Moreover, such a compact appropriately shaped device could be fitted easily within a vehicle, such for example within the trunk of a truck.

Considering that preferably the device of the first aspect of the invention is compact, preferably it has any of the following characteristics and combinations thereof:
- the device has a height of between 80 cm and 200 cm, and preferably of between 130 cm and 170 cm, and more preferably of between 145 cm and 160 cm, and most preferably of between 150 cm and 155 cm;
- the device has a length of between 50 cm and 180 cm, and preferably of between 90 cm and 150 cm, and more preferably of between 110 cm and 145 cm, and most preferably of between 130 cm and 140 cm, and/or is equal to the length of the main body;
- the device has a width that is of between 50 cm and 150 cm, and preferably of between 70 cm and 130 cm, and more preferably of between 80 cm and 110 cm, and most preferably of between 90 cm and 100 cm, and/or is equal to the width of the main body.
- the disinfection chamber has a height that is of between 80 cm and 200 cm, and preferably of between 130 cm and 170 cm, and more preferably of between 140 cm and 150 cm, and most preferably of between 140 cm and 145 cm, and/or is equal to the height of the main body;
- the disinfection chamber has a length that is of between 50 cm and 180 cm, and preferably of between 90 cm and 150 cm, and more preferably of between 100 cm and 125 cm, and most preferably of between 100 cm and 110 cm.
- the disinfection chamber has a width that is of between 50 cm and 150 cm, and preferably of between 70 cm and 130 cm, or of between 60 cm and 70 cm, and more preferably of between 90 cm and 120 cm, and most preferably of between 100 cm and 110 cm.

Moreover, preferably the device has at least four legs supporting on top of them the main body, wherein preferably each leg has a height of between 1 cm and 15 cm, and more preferably of between 4 cm and 10 cm. As such, the legs can keep the rest of the device and machinery above the surface or ground on which the device is placed, and protect the device and machinery from any potentially present water or dirt.

For maximizing compactness and ease of manufacturing, preferably the main body comprises a compartment that is adjacent to the disinfection chamber and contains within it any or both of the ozone generator and the ozone destroy means. The machinery contained in the ozone compartment is therein protected, and is also close to the disinfection chamber with which it communicates for passing or receiving ozone and/or other.

Optionally and preferably said compartment is separated from the disinfection chamber by a separation wall there between. This separation wall may be common between the two, with the compartment being one of the wall's facets, and the disinfection chamber being on the other of the wall's facets.

For improving the compactness and ease of manufacturing of the device, optionally and preferably the device has any of the following characteristics, and combinations thereof:
- the compartment has a height that is equal to, or is smaller or larger than, to the height of the disinfection chamber;
- the compartment has a length that is equal to, or is smaller or larger than the length of the disinfection chamber;
- the compartment has a width that is equal to, or is smaller or larger than, the width of the disinfection chamber.

Optionally and advantageously the main body comprises a service entrance configured to allow the user accessing the compartment. That way the user may easily service or replace the device's components that may be contained in the compartment. Likewise, said service entrance may contain openings that allow any heat that is produced by said components when the system operates, to be released to the environment so that said components are cooled and not overheated.

Optionally, the compartment is located behind the user command means. That way the length of any wiring required for connecting the control unit and/or the command means therein and the components controlled by said control unit, can be minimized, and thus the cost and complexity of the device can be reduced.

Optionally and preferably, said main body and said compartment have an exhaust opening wherein the end exhaust is located. Through said exhaust opening and end exhaust, the non-toxic gaseous product produced by the ozone destroy system that preferably is within said compartment, is quickly released to the environment soon after it is produced.

Optionally, at least part of said tubing system extends from the disinfection chamber to within the compartment. That way said disinfection chamber can communicate with the components in the compartment, and for example pass ozone to the ozone destroy system within the compartment. It is important to point out that the tubing system comprises tubes via which gases can pass between certain components of the device without uncontrollably be released to the environment, nevertheless said tubing system optionally does not comprise a continuous network of tubes. Therefore, the tubing system may comprise sections, each section having one tube or more tubes which may be interconnected and/or connected with the other components as described herein, and different sections may be located in different parts of the device without being directly interconnected with each other.

Optionally, the disinfection chamber is located in front of the compartment along the width of the main body. In a specific example, the user sees in front of him the door of the disinfection chamber, and behind said door and said chamber, there is the compartment containing the ozone destroy system. On the left of the disinfection chamber there is attached a second compartment wherein there is the ozone generator. Also on the left of the door and on the front side of said second compartment there are fixed the user command means which are accessible by the user.

Optionally and preferably said disinfection chamber and said compartment have in common two lateral vertical walls which are located opposite to each other across the length of the main body. Said walls for example can be normal to the lengthwise direction of the device and/or main body therein. In that case a first part of each facet of said wall would act as a lateral wall of the compartment, and a second part of said facet would act as a lateral wall of the disinfection chamber.

Optionally said main body has in common with the disinfection chamber and/or with the compartment a bottom floor and/or a top ceiling. This way the device is easy to fabricate.

Optionally and preferably the door has a transparent window configured to allow the user seeing within the disinfection chamber. This can allow the user to directly see whether there the chamber is sufficiently clean or is full, and act accordingly. This is particularly important when the device is to be used in busy establishments and the users must be able to act quickly when using or contemplating of using the device.

Optionally and preferably the main body has a control part that extends fully across the width and/or the height of the main body, the user command means and/or the control unit being located in said control part, and said control part being distinct from the disinfection chamber and/or the compartment. Moreover, the aforementioned second compartment is optionally located within said control part, and the ozone generation and/or the oxygen generator mentioned further below is also located therein.

Optionally and preferably the control part has a length that is of between 20 cm and 40cm, and preferably of between 30 cm and 35 cm. This way the compact of the device is improved, and likewise the device is easy to install.

Advantageously, optionally the control means and the door are both located on a side of the main body. This way a user can input the wearable in the disinfection chamber, and subsequently activate the device without having to move around it.

Advantageously the device is robust and does not brake easily. Likewise, the disinfection chamber and any other part of coming in contact with ozone gas does not get oxidized or deteriorate easily. For this reasons, optionally and preferably the interior of the disinfection chamber where the wearables are to be inserted is made of a material that preferably comprises any of an oxidation resistive material, a stainless steel, a chromium-nickel stainless steel, stainless steel 316, stainless steel doped with molybdenum and combinations therefrom.

Optionally the interior of the disinfection chamber (1a) in which the wearables are to be inserted has a volume of between 300 liters (litres) and 2500 liters, and preferably of between 800 and 1200 liters, and most preferably of between 880 and 920 liters. This volume matches very well the need to disinfect frequently several wearable items using a device (apparatus) that is not excessively large and can thus be transported to and be/or fitted easily in a store or in a hospital or other place of business or service, or in a vehicle.

Optionally the disinfection chamber comprises within it a rack configured for supporting on it wearables to be disinfected. Such a rack can be for example be used for placing on it face masks, or shoes or protective visors or other items or wearables that are inserted in the device to be disinfected. The rack is preferably made of or comprises steel or another material such as stainless steel or a material that is resistive to the ozone and can be cleaned easily.

Optionally and preferably the disinfection chamber comprises in its interior a hanger, which preferably is a beam that is supported from two opposing walls of the disinfection chamber and extends lengthwise across the latter, and said hanger is configured for hanging from the same wearables to be disinfected or bags or nets or containers containing said wearables. Likewise, optionally said hanger comprises an S-formed (S-shape) hook that is configured for hanging from the same wearables to be disinfected or bags or nets or containers containing said wearables. By hanging the items within the disinfection chamber, it is easier to disinfect them homogeneously.

Moreover, preferably the disinfection chamber is hermetically sealed when the door is closed, thusly preventing the escape of ozone from within it via any route other than through the tubing system connected to the disinfection chamber, when the system is operated. Hermetically sealed can be also understood as meaning that the chamber's walls and joints and connections therein with the tubing system or with the other components do not contain any undesirable cracks or holes via which ozone gas can leak towards outside the device when the device is operated, because ozone gas can be toxic if inhaled. Likewise, preferably the aforementioned main body is hermetically sealed when the door is closed, thusly preventing the escape of ozone from within it, when the system is operated.

Optionally and preferably the ozone generator comprises a precursor entrance via which a precursor gas enters the ozone generator when the latter generates ozone, the precursor gas preferably comprising ambient air or more preferably oxygen, and/or preferably wherein the ozone generator is a corona discharge ozone generator. Corona discharge ozone generators are reliable adding to the reliability of the device of the first aspect of the invention. Moreover, in the optional case wherein the precursor gas is ambient air, the ozone generator can directly draw (ambient) air and convert it, or part of it, to ozone.

Optionally the aforementioned precursor entrance communicates with a precursor opening at the main body, and via said precursor opening the precursor gas enters within the main body and within the ozone generator.

Optionally and preferably, said precursor gas comprises oxygen, and the device further comprises an oxygen generator that is configured to generate oxygen and supply said oxygen to the aforementioned precursor entrance. Optionally and preferably said oxygen generator comprises a zeolite molecular sieve (ZMS) configured to absorb nitrogen molecules from the air and thusly produce high purity oxygen of a preferred concentration of between 90% and 97%.

Optionally and preferably in the device the control unit is configured to control the oxygen generator. For example, the control unit is preferably configured to activate and deactivate, e.g. to power on demand, the oxygen generator, and/or to adjust the oxygen generator's oxygen generation rate. Likewise, the oxygen generator is preferably configured to be activated by the control unit, and for this purpose it may be connected via appropriate wiring with said control unit.

It is contemplated that optionally and preferably the aforementioned oxygen generator is configured to generate oxygen gas at rate of between 0.1 litre of oxygen per minute (L/min) and 100 L/min, and preferably of between 1 L/min and 10 L/min, and more preferably of between 4 L/min and 10 L/min, and most preferably the oxygen generator is configured to generate oxygen gas at rate of 10 L/min, when the device is operated. Said optional configurations offer the advantage of achieving the generation of enough oxygen used for ozone generation so that the device can function properly and within the operational parameters related to an efficient and fast disinfection.

Optionally and preferably the power supply is configured to supply electrical power of between 1 kW and 50 kW, and preferably of between 5 kW and 15 kW, and most preferably 10 kW. Said optional configuration can meet the power requirements of the device's various power consuming components when the device is operated. Likewise, optionally and preferably the power supply is configured to operate under and an electrical supply to the same of AC voltage of a value of 380 V or of 220 V and/or of frequency of 50Hz. Also optionally, the device comprises a 25 A circuit breaker.

Optionally and preferably the device of the first aspect of the invention comprises a cooling system configured to cool the ozone generator when the latter and the system are operated, the cooling system preferably being a water cooling system or an air cooling system, and wherein the control unit and the cooling system are configured so that the former controls the latter. The cooling system ensures that the ozone generator is not overheated as a result of its operation. Preferably the cooling system is preferably controlled by the control unit e.g. the control unit controls whether and/or when the cooling system is powered.

For accelerating and optimizing the disinfection it may be desirable to simultaneously treat the items and/or wearables to be disinfected with both ozone gas and ultraviolet radiation, especially UV-C radiation of a wavelength of between 100 nm and 280 nm. For this reason, optionally and preferably the device of the first aspect of the invention comprises an ultraviolet (UV) radiation source located within the main body, and preferably on or within the disinfection chamber, wherein the control unit is configured to control the operation of the UV radiation source, and the latter when operated is configured to generate and supply UV radiation within the disinfection chamber.

In the aforementioned case, optionally the UV radiation comprises any of UV-A, UV-B and UV-C, and combinations thereof, and preferably UV-C. The UV-A radiation has a wavelength of between 325 nm and 400 nm, and the UV-B radiation has a wavelength of between 280 nm and 315 nm.

In the aforementioned case, and for safety reasons, especially in the optional case wherein the door of the chamber comprises a transparent window such as glass window, optionally and preferably the door comprises a UV filter configured to block UV-C and/or UV-B and/or UV-A radiation to pass through said door and/or any transparent window thereon, when the door is closed.

The device of the first aspect of the invention optionally and preferably comprises a suction pump that is connected to the tubing system and is configured to be controllable by the control system when the system is operated, the control system being configured to control the suction pump, and wherein the suction pump and the tubing system are configured to do any of the following and combinations thereof when the system is operated:
- force ozone gas and/or other gases, for example atmospheric air and/or oxygen, to move out of the disinfection chamber and through the exhaust inlet and through the exhaust outlet and towards and/or through the ozone destroy system;
- evacuate, e.g. evacuate the gaseous atmosphere out of, the disinfection chamber;
- force atmospheric air and/or oxygen gas to move into and/or through the tubing system or parts thereof and/or into the disinfection chamber, preferably through the ozone outlet;
- force the non-toxic gaseous product to move out of the ozone destroy system and/or out of the system and/or through the end exhaust;
- force atmospheric air and/or oxygen gas to move into and/or through the tubing system and out of the ozone destroy system;
- force a precursor gas, such as for the example a precursor used by the ozone generator to produce ozone, to enter said ozone generator.

Most preferably the suction pump is configured to move ozone gas from the disinfection chamber to the ozone destroy system. Therefore, preferably the suction pump is located and connected at the tubing system in between the ozone destruction means and the disinfection chamber. The suction pump may be an electric fan. Preferably the control unit is connected via wire to the suction pump and configured to control the latter's powering and operation. When the suction pump operates while the ozone generator no longer generates and no longer supplies ozone to the disinfection chamber as is the preferred case when the disinfection is about to finish, the suction pump's action tends to create a vacuum within the disinfection chamber. To avoid creation of said vacuum, and to be able to clean said disinfection chamber from the ozone gas before the chamber can be opened for removing therefrom the disinfected items, optionally and preferably an air vent valve that preferably is a mechanical valve, is fixed on one of the walls of the disinfection chamber. Said air vent valve is configured to automatically open and allow atmospheric air from outside the main chamber to enter the latter when in said latter a vacuum tends to be created due to the action of the suction pump. Preferably the air vent valve is configured and/or graded to prevent the backflow through it of gas from the disinfection chamber to outside the latter.

Optionally and preferably, in said device of the first aspect of the invention, the aforementioned sensing means comprise a lock sensor configured to sense whether the door is fully closed or not, and the safety condition comprises or is that the door is fully closed. The lock sensor preferably is an electronic and connected via wire to the control unit, and the latter is configured to detect via the sensor whether the door is closed or open, and to prevent generation of ozone when the door is open so that a leak of ozone via the open door is avoided.

Optionally and preferably the sensing means comprise an external ozone sensor that is located outside the disinfection chamber and preferably outside the main body, and said external ozone sensor is configured to measure the concentration of ozone thereat, and the safety condition comprises or is that the said concentration of ozone measured by the external ozone sensor is less than a safe external concentration that preferably is of 0.05 ppm (parts per million). Optionally the external ozone concentration is connected via wire to the control unit, and both of them may be configured so that the latter controls the former and receives from it electric signals related to the measurements performed. If the ozone concentration measured by the external ozone is of a value that is larger than 0.05 ppm, then said concentration may be toxic to the user, and the control unit preferably automatically shuts down the ozone generator or the entire system. Obviously the external ozone sensor preferably is of the electronic type.

Optionally and preferably the sensing means comprise an internal ozone sensor that is located within the disinfection chamber or within the tubing system, and is configured to measure the concentration of ozone thereat, and preferably the safety condition is or comprises that the concentration of ozone measured by the internal ozone sensor is less than a maximum allowed internal concentration of preferably 10010 ppm or 4000 ppm or 3000 ppm or 2000 ppm. When the ozone concentration is larger than 10010 ppm, or is larger than one of the aforementioned maximum allowed concentration values, then the ozone may damage the items to be disinfected, and/or there may be an improper function of one of the device's components controlled by the control unit. Optionally the external ozone concentration is connected via wire to the control unit, and both of them may be configured so that the latter controls the former and receives from it electric signals related to the measurements performed. Obviously the external ozone sensor preferably is of the electronic type.

According to the above, optionally and preferably the sensing means are electronic and connected via wire to the control unit, and the sensing signal is an electrical signal transmitted via said wire, and the control unit and the sensing means are configured so that the control unit can detect and/or process said electric signal and/or can control the sensing means.

Optionally and preferably in the device the ozone destroy system comprises a catalytic ozone destructor comprising a catalyst wherein preferably the catalyst comprises manganese oxide and/or copper oxide. In a preferred but not limiting example, the catalyst comprises or is a CARULITE® 200 catalyst.

Moreover, optionally and preferably the ozone destroy system comprises a deodorization system, such as for example a deodorization filter, that is configured to deodorize or aromatize the non-toxic gaseous product. In a non-limiting contemplated example, the aforementioned deodorization filter is an active carbon filter, and the ozone destroy system comprises the deodorization filter connected in series with a catalytic ozone destructor, and the system is configured so that the gasses processed by the ozone destroy system first are processed by the catalyst filter and subsequently are processed by the deodorization filter. A deodorization filter is preferably included when the system is destined to be used within a cloth store wherein any the release by the system of unpleasant smells is to be avoided.

Optionally and preferably the device according to the first aspect of the invention comprises a ventilation system configured to homogenize the ozone gas concentration within the disinfection chamber when the system and the ozone generator are operated. Essentially the optional ventilation system is destined to force the ozone gas to disperse through the volume of the disinfection chamber and flow therein so the surfaces of the items being disinfected are well treated homogenously and continuously.

When the device comprises the aforementioned ventilation system, then preferably said ventilation system comprises an electric fan located in the disinfection chamber or in or connected to the tubing system, and is configured to increase the travel speed or flow of any gas, such as ozone, that enters via the ozone outlet or is within the disinfection chamber. Preferably the ventilation system comprises two or more electric fans. Said optional ventilation system and the control unit are preferably configured so that the latter controls the former.

Optionally and preferably in a system according to the first aspect of the invention the user command means comprise a power button or knob and the like configured for switching on or off the power supply. In this optional case, the user can switch on and switch off the power supply and the system by handling said power button or knob.

Moreover, optionally and preferably the user command means comprise an emergency button configured for stopping all operation of the device without interrupting the power supply of the same. In this optional case, the user can stop all operation of the device without interrupting the power supply of the same by handling said emergency button. Moreover, optionally and preferably the user command means comprise a reset button configured for restarting operation of the system or of the control unit or of the computer therein in case of a failing. Preferably for security reasons said reset button has to be used by the user every time the device is switched on.

Optionally and preferably the user command means comprise an electronic touchscreen. Likewise, optionally and preferably the user control means are configured to display to the user several operation command options and sense a touching or selection of any of said operation command options by the user, and the computer is configured to convert said touching or selection of any of said operation command options to a corresponding operation command that is pre-stored and/or programmed in the computer. The computer may be PLC. In a non-limiting example related to preferred embodiment of the device according to the first aspect of the invention, the electronic operation command options are displayed via a touchscreen, and comprise icons related to activating or deactivating, such as powering on or off, any of the system's components, such as the ozone generator or ozone destroy system. More preferably an electronic command option relates to the coordinated activation and deactivation and operational control by the control unit of the device's components for thusly executing with the device a disinfection cycle. The disinfection cycle includes checking whether the safety condition is met and executing, preferably in a consecutive manner, the following: activating the ozone generator and the ventilation system for generating passing and dispersing ozone to/in the disinfection chamber for a desired period of time; deactivating the ozone generator and activating the ozone destroy system and the suction pump for removing ozone from the disinfection chamber and filling said chamber with atmospheric air until the ozone has been completely or nearly completely removed from the disinfection chamber; stopping the system and indicating to the user that the disinfection chamber has been completed. The system preferably comprises an electric locking mechanism that is connected to and/or is controlled by the control unit, and said locking mechanism is configured to lock the door while a disinfection cycle occurs and/or while the ozone generator is operated. Therefore, an operation command option may include keeping activating said locking mechanism for a specific period of time or while certain conditions exist. Each operation command option preferably is related to a corresponding command that is pre-stored and/or programed to the computer or PLC. The optional touchscreen and the computer or PLC connected to said touchscreen can optionally be configured so that via the touchscreen the user may program the disinfection cycles, or may activate and/or control individual components of the system.

Optionally and preferably the tubing system comprises an air vent with an air vent valve therein configured to let air enter the tubing system and/or the disinfection chamber when the air vent valve is open, wherein said air vent valve is controllable by the control unit or is mechanical. The air vent valve can be considered to be similar to the air vent valve described further above.

Optionally and preferably one of said pre-stored and/or programmed in the computer operation commands comprises any of the following or combinations thereof or preferably all of:
- activate the oxygen generator for generating oxygen;
- activate the ozone generator for generating ozone gas for a total duration of between 1 second and 20 minutes, or for an ozone generation duration of between 1 second and 10 minutes (min), or for a disinfection duration of between 30 seconds (s) and 20 min, and preferably of between 30 seconds and 5 min, while communicate and measure with the internal ozone sensor an ozone concentration of between 1 ppm and 10000 ppm, and preferably 1 ppmand 2000 ppm, preferably 1 ppm and 1000 ppm or preferably of between 1 ppmand 700 ppm;
- while generating with the ozone generator ozone gas, activate and operate the cooling system for using the same to cool the ozone generator;
- while generating with the ozone generator ozone gas, activate thusly using the ventilation system to homogenize the ozone gas concentration within the disinfection chamber;
- after the disinfection duration ozone generation duration has passed, deactivate or shut down or stop operating the ozone generator and/or the oxygen generator and/or the cooling system and/or the ventilation system, and activate and operate the pump to remove ozone from the disinfection chamber, and open the air vent valve when the latter is controllable by the control unit, and activate and operate the ozone destroy system thusly destroying ozone gas until the internal ozone sensor measures an ozone concentration of less than 0.05 ppm and/or until a safety period of time has passed;
- when the internal ozone sensor measures an ozone concentration of less than 0.05 ppm, and/or when the safety period of time has passed, switch off the pump and preferably close the air vent valve when the latter is controllable by the computer;
- indicate to the user via the touchscreen that the operation command has been executed;
- increasing the temperature inside the disinfection chamber to the desired disinfection temperature, using the heating system.

Optionally and preferably the wearables are any of clothes, apparel, clothing accessories, trousers, shoes, hair caps, personal protective equipment (PPE) such as protective clothing, helmets, goggles, face masks or other garments or equipment designed to protect the wearer's body from injury or infection, and combinations thereof.

It is contemplated the optional and preferable case wherein the system according the first aspect of the invention further comprises any or both of:
- a humidity sensor located within the disinfection chamber or attached to or within the tubing system and configured to measure there at the humidity concentration and generate and transmit a corresponding humidity signal;
- a humidity control system configured to control the humidity within the disinfection chamber by introducing humidity, or removing humidity from, within the disinfection chamber;
   wherein preferably the control unit is configured to control the humidity sensor and receive said humidity signal, and/or is configured to control the operation of the humidity control system. The humidity control system is configured to be controlled by the control unit. The control unit is preferably configured to operate the humidity control system to cause that the humidity concentration measured by the humidity sensor acquires a preferred humidity concentration value. The preferred humidity concentration value preferably is of between 8% and 80%, and more preferably of between 10% and 70%, and most preferably of between 10% and 40%. Achieving said preferred humidity concentration values may result to improving the efficacy and efficiency of the disinfection achieved with the system because water is known to affect the physiochemical effects of ozone treatment on surfaces. Likewise, the humidity value can be adjusted considering the specific items to be disinfected and the materials therein. The humidity control system may comprise a humidifier.

Moreover, it is contemplated the optional and preferred case wherein the operational command and/or the related operation command option is related to the material or type of the items or wearables to be disinfected, or the quantity or volume of the items to be disinfected. For example, an operation command may include specifying a type of item or wearable or characteristic therein such as a material of the wearable, and/or adjusting a recipe parameter according to said specifying, said recipe parameter being any of the following and combination thereof:
- Disinfection time;
- Ozone generation rate;
- Preferred Humidity value or range;
- maximum allowed internal concentration of ozone (as measured by an internal ozone concentration sensor);
- ozone concentration value or value range as measured by an internal ozone sensor;
- ozone generation duration;
- safety period of time
- desired disinfection (sanitization) temperature
- Heating duration.

For example the operation command may comprise accessing a computer file according to which an initial condition, said condition being any of a specific material to be disinfected, or an item to be disinfected, or a volume or quantity of items to be disinfected, or a specific level of disinfection required, or a specific protocol of disinfection required, is correlated with at least one value or value range of any of the aforementioned recipe parameters and combinations thereof, and controlling the system and components thereof as required to realize as part of implementing a disinfection cycle the recipe parameter value or value range that correlates to the initial condition. The computer file may be stored to the computer (or PLC) or a storage medium attached therewith or a remote server connected to said computer.

A second aspect of the invention concerns a vehicle that comprises a device that is according to the first aspect of the invention. Said device of the vehicle may comprise any of the aforementioned optional elements and related configurations. The vehicle preferably is a truck such as a station wagon, or a pickup truck or a lorry. In said vehicle the device is preferably located on the trunk (boot) of said vehicle and configured there so that a user can use the device for disinfecting items such as wearables. Likewise, preferably the vehicle is configured for powering the power unit of the device. For example, optionally the electrical system of the vehicle is configured to power the device. Likewise, the vehicle optionally has a separate electrical power generator that is configured to power the device. Likewise, optionally the vehicle has batteries and is configured to power with said batteries the device. The vehicle and the device located on/in it may be considered as being the two essential parts of a system. Said system, i.e. the vehicle with the device on it, solves the technical problem of how to serve the needs of different establishments wherein there exist items that need to be disinfected, especially when said establishments do not have the required facilities for disinfecting the items, and especially when due to a high temporal spike in the establishment's workload said establishments require to promptly use frequently, e.g. daily, the services of a disinfection facility. This can occur for example when the establishments are hospitals and there is an epidemic or a pandemic outbreak and said hospitals receive many infected patients, and thusly the hospitals require to use daily or very often an external disinfection facility for disinfecting items such as personal protection equipment items, e.g. face masks, used by the hospital's personnel. The vehicle, i.e. the vehicle-device system, of the second aspect of the invention is the disinfection facility, and solves exceptionally the above problem because it is a mobile facility that can visit the hospital. Likewise, said vehicle (system) due to the transportability, safety and efficiency of the device which can be used for disinfecting items in a fast, efficient, safe and well controlled manner, can visit and serve several facilities or hospitals within a single day. This can help tremendously health organizations, such as public health systems that comprise several hospitals and health centers scattered across several places and/or regions. Likewise, in case of an emergency in a specific region or country, a fleet of the vehicle-device systems can be quickly dispatched to said specific region or country to assist with the emergency.

Therefore, preferably the invention in its second aspect concerns a truck for disinfecting wearables, the truck comprising a device that is according to the first aspect of the invention and is located in/on a trunk of said truck, the truck preferably being configured to supply sufficient power to the power supply unit of the device for powering and operating the latter.

The invention its third aspect concerns a method for using said vehicle or truck for disinfecting items such as wearables. Therefore, the invention in its third aspect describes (concerns) a method for disinfecting wearables, particularly personal protective equipment used in a facility, the method comprising:
- driving a truck that is according to the second aspect of the invention towards the facility that may be a healthcare establishment;
- moving the wearables from the facility to the truck;
- disinfecting the wearables using the truck and the device therein;
   and the method preferably comprises also,
- moving the disinfected wearables from the truck to the facility;

Likewise, the invention in its fourth aspect concerns/describes a method for disinfecting wearables, particularly personal protective equipment, used in a first and a second or additional facilities, the method comprising:
- driving a truck that is according to the second aspect of the invention towards the first facility that may be a first healthcare establishment;
- moving wearables from the first facility to the truck;
- disinfecting the wearables using the truck and the device therein;
- moving the disinfected wearables from the truck to the first facility;
- driving the truck towards the second facility that may be a second healthcare establishment;
- moving wearables from the second facility to the truck;
- disinfecting the wearables using the truck and the device therein;
- moving the disinfected wearables from the truck to the second facility.

The invention its fifth aspect concerns a method for disinfection items, such as wearables, and in particular personal protective equipment items such as for example face masks.

Preferably said method of the fifth aspect of the invention comprises:
- moving and placing the item to be disinfected to within a disinfection chamber of a system that optionally and preferably is a system according to the first aspect of the invention and/or comprises the disinfection chamber that has a door via which the items are being placed, and an ozone generator, an ozone destroy system and a control a control unit;
- closing the door;
- generating ozone gas using the ozone generator and supplying said ozone gas to the disinfection chamber;
- passing said ozone gas from the disinfection chamber and converting said ozone gas to a non-toxic gaseous product, and preferably releasing said non-toxic gaseous product;
- opening the door and removing the items from the disinfection chamber.

The items preferably are wearables, and more preferably are wearable personal protection equipment (PPE) such as for example face masks.

Optionally and preferably moving and placing the items to be disinfected comprises:
- placing the items in a receptacle, wherein said receptacle preferably is a bag, and more preferably is a net bag, i.e. string bag, and the like;
- moving said receptacle to within the chamber.

Preferably moving said receptacle comprises avoiding touching the contents of the receptacle.

Likewise removing the items may optionally comprise avoiding touching the contents of the receptacle.

In the aforementioned case wherein the items have been placed in receptacle, removing the items may optionally comprise avoiding touching the receptacle (or/and the items within the receptacle).

By not touching the receptacle or the contents of the receptacle a good safety and hygiene (sanitation) protocol related to the disinfection method is established.

Likewise, the receptacle or bag preferably has a hook, such an S-shaped hook attached to it. For this reasons moving and placing the items to be disinfected may optionally further comprise:
- attaching an S-hook to the bag;
- holding the S-hook avoiding touching the bag and its contents; and optionally,
- hanging said S-hook within the disinfection chamber.

Likewise removing the items from the disinfection chamber may comprise any of the following:
- holding the S-hook avoiding touching the bag and/or its contents (the items/wearables);
- un-hanging the S-hook from within the disinfection chamber.

Preferably, the disinfection chamber has at least one hanger configured so that the bag or an S-hook with said bag can be hanged from said hanger.

Moving the item to be disinfected may optionally comprise any of the following and combinations thereof:
- within a first facility such as for example a healthcare establishment such as for example a hospital, placing the items within a first package that for example is a first parcel or a sealable plastic bag;
- transporting said first package to a second facility or vehicle that contains the disinfection chamber;
- removing the items from the first package.

Likewise, the method of the fifth aspect of the invention, after removing the disinfected items from the chamber optionally comprises any of the following and combinations therefrom:
- placing the items or a receptacle containing them within a second package that for example is a second parcel or a sealable plastic bag, while preferably avoiding touching the items or a receptacle containing them;
- placing a sign on said second parcel indicating that the items are disinfected;
- transporting said second package to the first facility.

Preferably said method of the fifth aspect of the invention comprises:
- wearing personal protective equipment while applying the method.

In the method of the fifth aspect of the invention, closing the door preferably comprises locking the door.

Likewise, generating ozone gas using the ozone generator optionally comprises generating ozone gas at a rate of between 1 grams of ozone per hour to 100 grams of ozone per hour, and preferably of between 1 grams of ozone per hour to 60 grams of ozone per hour and supplying said ozone gas to the disinfection chamber.

Likewise, generating ozone gas using the ozone generator and supplying said ozone gas to the disinfection chamber is optionally performed for an ozone generation duration of between 1 second and 10 minutes (min) or preferably of between 30 seconds and 5 min, or for a disinfection duration of between 1 second (s) and 20 min, and preferably of between 30 seconds and 10 min,

Likewise, optionally the method comprises supplying said ozone gas to the disinfection chamber at an ozone concentration of between 1 ppm and 10000 ppm or preferably of between 1 ppm and 2000 ppm as measured by an ozone sensor thereat, e.g. using an internal ozone sensor located at the disinfection chamber or at a tubing system that connects the ozone generator with the disinfection chamber and via which the ozone gas is being supplied.

Likewise, the method optionally and preferably comprises stopping passing (supplying) said ozone gas to the disinfection chamber, and converting said ozone gas to a non-toxic gaseous product until the ozone concentration within the disinfection chamber as measured by said internal ozone sensor is less than 0.05 ppm, or until a safety period of time has passed.

Likewise, the method optionally comprises, while supplying said ozone gas to the disinfection chamber, controlling the humidity within the disinfection chamber by introducing humidity, or removing humidity from within the disinfection chamber. Optionally and preferably controlling the humidity comprises causing that the humidity concentration within a chamber acquires a preferred humidity concentration value which preferably is of between 8% and 80%, and more preferably of between 10% and 70%, and most preferably of between 10% and 40%.

Any of the actions caused by the components of the device of the first aspect of the invention as described further above in relation to the operation of said device, and any conditions or properties related to said components or operation, should also be considered as an optional corresponding element of the method according to the fifth aspect of the invention.

In a method according to the third or the fourth or the fifth aspect of the invention preferably the facility or the first facility or the second facility, is any of a healthcare facility, a hospital, a medical center, a public service building, a school, a factory, a store, a laundry shop, a goods storage facility, a textile processing facility or factory, a food production or processing facility or open field; likewise, the wearables are any of clothes, apparel, trousers, shoes, hair caps, personal protective equipment (PPE) such as protective clothing, helmets, goggles, face masks or other garments or equipment designed to protect the wearer's body from injury or infection, and combinations thereof.

### Brief Description of Drawings

The previous and other advantages and features will be more fully understood from the following detailed description of embodiments, with reference to the attached figures, which must be considered in an illustrative and non-limiting manner, in which:
FIG 1 is a schematic diagram of a preferred embodiment of a device (apparatus) according to the first aspect of the invention.
FIG 2 is a schematic diagram of a part of a preferred embodiment of a device (apparatus) according to the first aspect of the invention, showing how some important components of the device are arranged.
FIG 3 is a schematic diagram of a part of another preferred embodiment of a device (apparatus) according to the first aspect of the invention, showing how some important components of the device are arranged.
FIG 4a is a front view (view from the front) schematic diagram of a preferred embodiment device (apparatus) according to the first aspect of the invention, wherein the device is shaped as benchtop instrument.
FIG 4b is a side view (view from the side) schematic diagram of the preferred embodiment of Fig 4a.
Fig 4c is a top view (view from the top) schematic diagram of the preferred embodiment of Fig 4a.
FIG 5 is a perspective drawing of a preferred embodiment device (apparatus) according to the first aspect of the invention, wherein the device is shaped as a closet, wherein a door of the disinfection chamber is closed.
FIG 6 is a more detailed drawing from the same perspective and of the same device shown in FIG 5.
FIG 7 is a detailed drawing from a different front-view perspective of the same device shown in FIG 6, but with the door of the disinfection chamber being open, and also a service entrance being open.
FIG 8. is a detailed drawing from a side-view perspective of the same device shown in FIG 7.
FIG 9 is a perspective drawing of a preferred embodiment device that is shown in FIG 4a, FIG 4b and FIG 4C.
FIG 10a is a more detailed drawing of the same device and from the same perspective shown in FIG 9.
FIG 10b is a drawing of the same device but from a back-view perspective of the device shown in FIG 10a.
FIG 11 is a drawing from a back-view of a vehicle (vehicle-device system) according to the second aspect of the invention, wherein the vehicle is a truck. The arrow points to the truck.
FIG 12a is a drawing of an S-shaped hook used with the device according an embodiment of a method according to the third, fourth or fifth aspect of the invention.
FIG 12b is a drawing of a string bag (net bag) used with the device according an embodiment of a method according to the third, fourth or fifth aspect of the invention.
FIG 13 is a schematic diagram of a part of a preferred embodiment of a device (apparatus) that comprises all the elements of the device of Fig. 2, and further comprises a heating system.

### Detailed Description of Preferred Embodiments

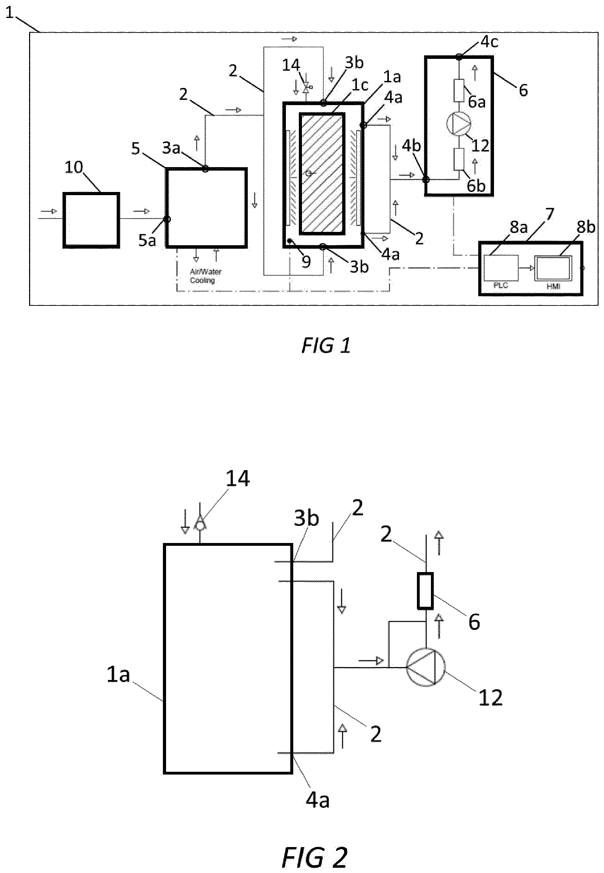

FIG 1 is a schematic diagram of a preferred embodiment of a device (apparatus) according to the first aspect of the invention; in said embodiment and as shown in FIG 1, the device comprises:
- a main body 1 that has a height (not indicated), a length (not indicated) and a width (not indicated), and comprises a disinfection chamber 1a which has a door 1c and is configured for receiving within it and via the door 1c the wearables to be disinfected;
- a tubing system 2 that has an ozone inlet 3a, an ozone outlet 3b, an exhaust inlet 4a, an exhaust outlet 4b and an end exhaust 4c, wherein the tubing system 2 is connected to the disinfection chamber 1a via the ozone outlet 3b and the exhaust inlet 4a, and is configured to, during an operation of the device by a user, supply to the disinfection chamber 1a ozone gas via the ozone outlet 3b, and to receive from the disinfection chamber 1a ozone gas via the exhaust inlet 4a;
- an ozone generator 5 connected to the tubing system 2 and configured to generate ozone gas at a rate of between 1 grams of ozone per hour to 100 grams of ozone per hour, and preferably of between 1 grams of ozone per hour to 60 grams of ozone per hour, and supply via the ozone inlet 3a said ozone gas to the tubing system 2;
- an ozone destroy system 6 connected to the tubing system 2 and is configured to receive from the latter via the exhaust outlet 4b ozone gas that has been received by the tubing system via the exhaust inlet 4a, and to convert said ozone gas to a non-toxic gaseous product, and to supply said non-toxic gaseous product to the end exhaust 4c;
- sensing means 9 configured for sensing at least one operational parameter and generating and transmit a corresponding sensing signal;
- a control unit 7 that comprises a power supply for powering the device, a computer 8a and user command means 8b configured for receiving operation commands from a user, the computer being a programmable logic controller, and the control unit 7 is configured to control the operation of the device according to the operation commands, and is configured to receive the sensing signal and inhibit the operation of the system when the sensing signal does not correspond to a safety condition. Some of the wired connections between the control unit and other components are represented in FIG 1 with dashed-dotted lines.

FIG 1 also shows that in the device the ozone generator 5 comprises a precursor entrance 5a via which a precursor gas enters the ozone generator 5 when the latter generates ozone. FIG 1 also shows that the device further comprises an oxygen generator 10 that is configured to generate oxygen and supply said oxygen to the precursor entrance 5a, and said precursor gas comprises oxygen, the oxygen generator comprising a zeolite molecular sieve (ZMS) configured to absorb nitrogen molecules from the air thusly producing high purity oxygen of preferred concentration of between 90% and 97%. The indicated in FIG 1 sensing means 9 comprise an external ozone sensor, and the indicated user command means comprise a touchscreen.

As shown in FIG 1 the device comprises a suction pump 12, that is connected to the tubing system 2 and is configured to be controllable by the control system 7 when the system is operated, the control system 7 being configured to control the suction pump 12, and wherein the suction pump 12 and the tubing system 2 are configured to:
- force ozone gas and/or other gases, for example atmospheric air and oxygen, to move out of the disinfection chamber 1a and through the exhaust inlet 4a and through the exhaust outlet 4b and towards and through the ozone destroy system 6;
- evacuate, e.g. evacuate the gaseous atmosphere out of, the disinfection chamber 1a;
- force the non-toxic gaseous product to move out of the ozone destroy system 6 and through the end exhaust 4c.

In some embodiments the suction pump 12 is part of the ozone destroy system.

As indicated in FIG 1, in the system depicted the tubing system 2 comprises an air vent with an air vent valve 14 therein configured to let air enter the disinfection chamber 1a when the air vent valve is open, wherein said air vent valve 14 is mechanical; the indicated air vent valve is configured to open when the suction pump is activated and draws gases from the disinfection chamber.

According to FIG 1 the ozone destroy system 6 comprises a catalytic ozone destructor 6b comprising a catalyst; the catalyst in that particular embodiment comprises manganese oxide and copper oxide. Likewise, according to FIG 1 the ozone destroy system 6 shown comprises a deodorization system 6a; the deodorization system 6a in that particular embodiment comprises a deodorization filter that comprises active carbon and sepiolite. As indicated in Fig 1 the device further comprises a cooling system (not fully shown) configured to cool the ozone generator when the latter and the system are operated.

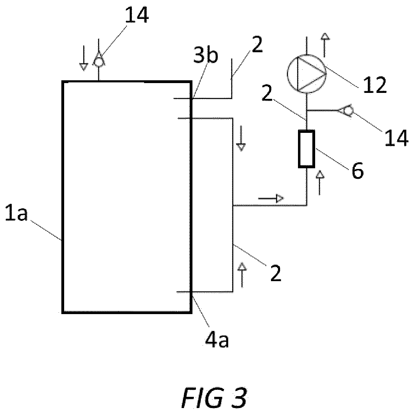

In FIG 1 the arrows show the main flow directions of gases via the system's components when the system is operated. The same applies for FIG 1 and FIG 3.

While in the embodiment of Fig 1 the suction pump 12 is located in between the deodorization system (carbon and sepiolite filter) 6a and the ozone destructor (a filter comprising CAROULITE®), in other embodiments as the one partially described in FIG 2, the suction pump is located/connected in between the ozone destroy system 6 and the disinfection chamber 1a. Fig. 3 describes part of yet another embodiment of a device, wherein however the suction pump 12 is located above/after the ozone destroy system, and in between the two there is an air vent valve 14 connected to the tubing system and configured to let air enter the tubing system when the suction pump is activated.

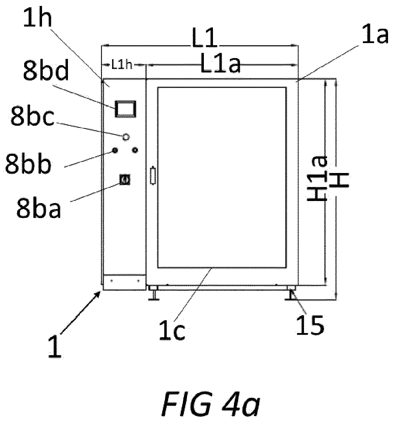

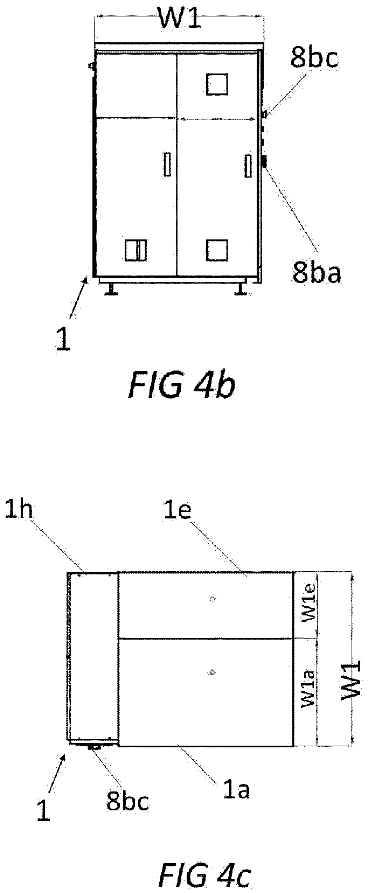

FIG 4a, FIG 4b and FIG 4c schematically describe a preferred embodiment of the device, wherein the device has the shape and form of an instrument that can easily be used on a workbench, and due to its compact size and overall configuration can easily be transported and used; specifically this embodiment has the following properties:
- the user command means comprise a power button 8ba or knob and the like configured for switching on or off the power supply;
- the user command means comprise an emergency button 8bb configured for stopping all operation of the device without interrupting the power supply of the same;
- the user command means comprise a reset button 8bc configured for restarting operation of the system or parts therein in case of a failing, and preferably for security reasons said reset button has to be used by the user every time the device is switched on;
- the user command means comprise an electronic touchscreen 8bd configured to display to the user several operation command options and sense a touching or selection of any of said operation command options by the user, and the computer is configured to convert said touching or selection of any of said operation command options to a corresponding operation command that is pre-stored and/or programmed in the computer;
- the main body 1 comprises a compartment 1e that is adjacent to the disinfection chamber 1a and contains within it the ozone destroy means (not shown);
- the main body 1 has a control part 1h that extends fully across the width W1 a and height H1a of the main body 1, the user command means being located in said control part 1h, and said control part 1h being distinct from the disinfection chamber 1a and the compartment 1e;
- the device's main body 1 has a length L1 a width W1, a height H1 (not indicated), the device has a length height H, the compartment 1e has a width W1e, the main chamber 1a has a height H1a and a width W1a, wherein in this particular embodiment:
   ∘ L1=1358 mm
   ∘ H = 1523 mm
   ∘ L1a= 1050 mm
   ∘ H1a= 1418 mm and is about equal to H1
   ∘ L1h= 308 mm
   ∘ W1= 1050 mm
   ∘ W1a= 650 mm
   ∘ W1e= 400 mm

In another embodiment shaped as a closet the dimensions are as follows:
L1= 1700 mm
W1= 1100 mm
H= 1890 mm

In another embodiment shaped as benchtop instrument the dimensions are as follows:
L1 = L=1325 mm
W1= L = 1225 mm
H= 1540 mm

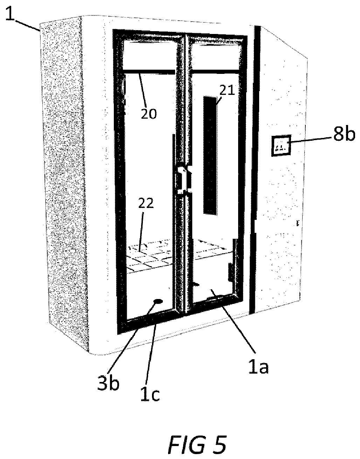

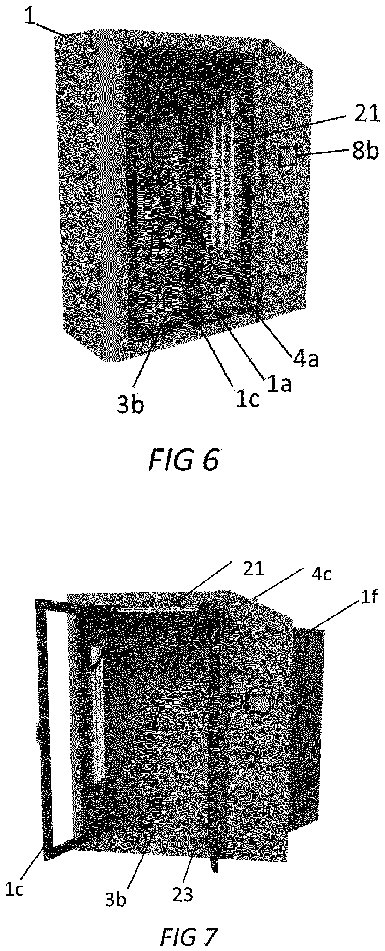

FIG 5, FIG 6, FIG 7 and FIG 8 show an embodiment wherein the device is shaped as a closet so that the main chamber 1a has about the height of, or is slightly taller or shorter than, an average user so that said average user can open the door 1c and place or hang inside the main chamber 1a some clothes to be disinfected. The particular embodiment is contemplated to primarily be easily installed and used in existing cloth stores. As shown by FIG 5 and FIG 6:
- the disinfection chamber 1a comprises in its interior a hanger 20, which is a beam that is supported from two opposing walls of the disinfection chamber 1a and extends lengthwise across the latter, and said beam (hanger) is configured for hanging from the same wearables to be disinfected or bags or nets or containers or other hangers containing said wearables;
- the user command means comprise a touchscreen that is located next to the door 1c and on the same side as the door 1c,
- ozone outlets 3b are located at the floor of the disinfection chamber 1c;
- the device comprises an ultraviolet UV radiation source 21 located within the main body 1 and within the disinfection chamber 1a, wherein the control unit (not shown) is configured to control the operation of the UV radiation source, and the latter when operated is configured to generate and supply UV radiation within the disinfection chamber 1a;
- the disinfection chamber 1a comprises within it a rack 22 configured for supporting on it wearables to be disinfected.

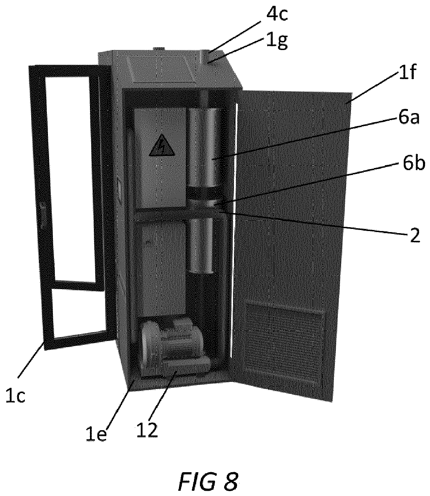

As is further shown in FIG 7 and FIG 8 which shows the same embodiment with the door 1c being open:
- there are also some UV radiation sources 21 (tube UV lamps in this particular case) at the ceiling of the disinfection chamber;
- the main body 1 and the compartment 1e have an exhaust opening 1g wherein the end exhaust 4c is located;
- the main body comprises a service entrance 1f (shown here being open) configured to allow the user accessing the compartment 1e;
- the device comprises a ventilation system 23 configured to homogenize the ozone gas concentration within the disinfection chamber when the system and the ozone generator are operated, and in this particular embodiment the ventilation system 23 comprises electric fans being on the floor of the disinfection chamber 1a.

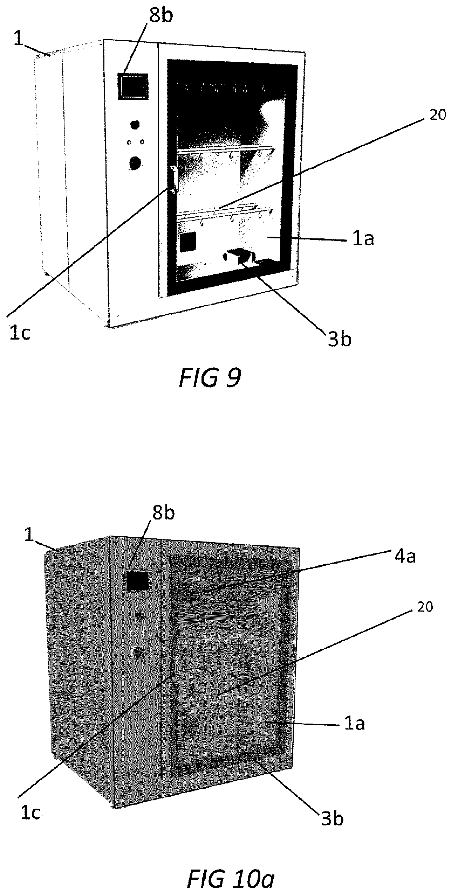

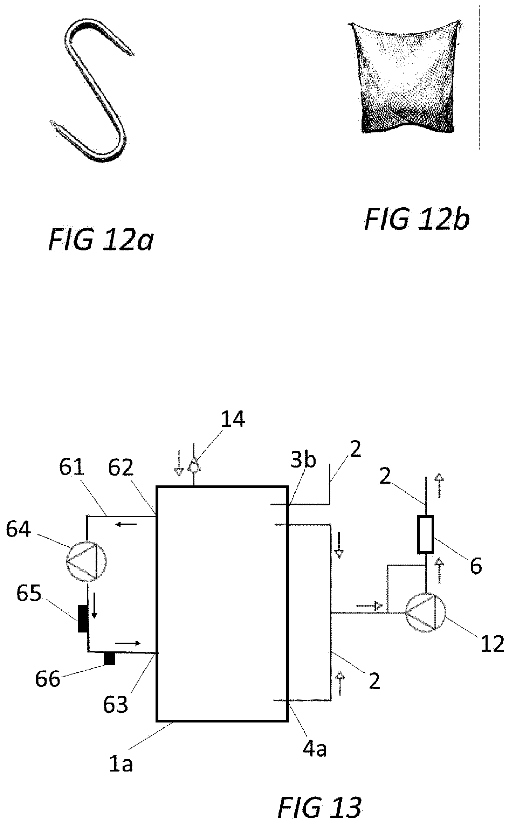

The device described further above in relation to FIG 4a-4c may also comprise a hanger 20 as shown in FIG 9 and FIG 10a. Said hanger can for example be used for hanging from the same S-shaped hooks, as for example the hook shown in FIG 12a, and string (net) bags as for example the one shown in FIG 12b. In said bags other items/wearables can be placed for being disinfected with the device. The string bags are preferably being attached to said hooks.

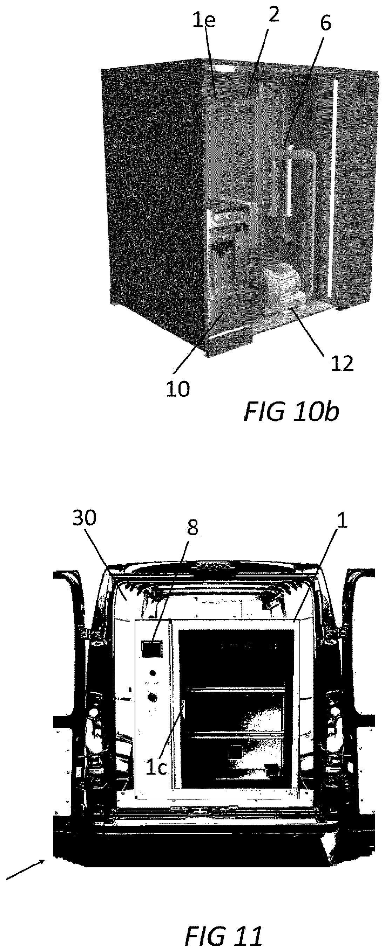

FIG 10a further shows an exhaust inlet 4a via which ozone can travel via the tubing system towards the ozone destroy system. FIG 10b shows a part of said tubing system 2 located in the compartment 1e. In the latter there are also located a suction pump 12, the ozone destroy system 6 and an oxygen generator 10.

FIG 11 shows a back view of a truck that is a preferred embodiment of the third aspect of the invention. The truck is a pickup truck and is shown with its backdoor being open. As shown in FIG 11 within the trunk of the truck there is a device according to the embodiment shown in FIG 9, FIG 10a ad FIG 10b. The device due to its compact size and advanced configuration fits very well within the pickup track and is positioned in a way that makes it easy to be used without requiring be moved further within the truck. In the shown example, a user standing on the ground can open the truck's backdoor and directly use the device without having to jump in the trunk.

The device of Fig. 13 comprises a heating system, and more specifically Fig. 13 shows a case of a device (system) according to the first aspect of the invention, wherein the tubing system 2 further comprises a tubing circuit 61 that has a circuit inlet 61 and a circuit outlet 63, said tubing circuit 61 being connected to the disinfection chamber 1a via the circuit inlet 62 and the circuit outlet 63, and the device further comprises a heating system that when operated is configured to increase a temperature inside the disinfection chamber 1a, the heating system comprising a heating element 65, an auxiliary suction pump 64, and a temperature sensor 66, the heating element 65 when operated being configured to heat inside the tubing circuit 61, the auxiliary suction pump 64 when operated being configured to force any gas from inside the disinfection chamber 1a to successively exit said disinfection chamber 1a via said circuit inlet 62, flow inside said tubing circuit 61, and reenter the disinfection chamber 1a via the circuit outlet 63. The direction of the flow of the gases inside the tubing circuit under the action of the auxiliary suction pump, is indicated by the black arrows thereat in Fig 13. In the system shown in Fig. 13 both the heating element and the temperature sensor are installed at the tubing circuit.

A method according a preferred embodiment of the third aspect of the invention comprises:
- driving the truck similar to the one shown in FIG 11 towards a hospital;
- moving PPE (personal protection equipment) wearables from the hospital to the truck;
- disinfecting the PPE wearables using the truck and the device therein;
- moving the disinfected wearables from the truck to the hospital.

A method according to a preferred embodiment of the fourth aspect of the invention comprises:
- driving the truck similar to the one shown in FIG 11 towards a first hospital;
- moving PPE wearables from the first hospital to the truck;
- disinfecting the PPE wearables using the truck and the device therein;
- moving the disinfected PPE wearables from the truck to the first hospital;
- driving the truck towards a second hospital;
- moving wearables from the second hospital to the truck;
- disinfecting the PPE wearables using the truck and the device therein;
- moving the disinfected PPE wearables from the truck to the second hospital.

A method according to a preferred embodiment of the fifth aspect of the invention comprises:
- moving and placing the item (or items) to be disinfected to within a disinfection chamber of a system that is a device as the one shown in FIG 9 or a truck as the one shown in FIG 11 and comprises a disinfection chamber that has a door via which the items are being placed, an ozone generator, an ozone destroy system and a control unit;
- closing the door;
- generating ozone gas using the ozone generator and supplying said ozone gas to the disinfection chamber;
- passing said ozone gas from the disinfection chamber and converting said ozone gas to a non-toxic gaseous product, and preferably releasing said non-toxic gaseous product;
- opening the door and removing the items from the disinfection chamber.

A method according to another preferred embodiment of the fifth aspect of the invention is a method for sanitizing a wearable or another item, the method comprising:
- moving and placing the wearable to be sanitized within a disinfection chamber of a system that is according to a preferred embodiment of the first aspect of the invention, and comprises a disinfection chamber that has a door via which the items are being placed, a heating system as the one of the device in Fig. 13, an ozone generator, an ozone destroy system and a control unit;
- closing the door;
- increasing the temperature inside the disinfection chamber using the heating system.
- generating ozone gas using the ozone generator and supplying said ozone gas to the disinfection chamber;
- passing said ozone gas from the disinfection chamber and converting said ozone gas to a non-toxic gaseous product, and releasing said non-toxic gaseous product;
- opening the door and removing the items from the disinfection chamber.

### CLAUSES

CLAUSE 1. A device for disinfecting wearables, comprising:
   - a main body (1) that has a height (H1), a length (L1) and a width (W1), and comprises a disinfection chamber (1a) which has a door (1c) and is configured for receiving within it and via the door (1c) the wearables to be disinfected;
   - a tubing system (2) that has an ozone inlet (3a), an ozone outlet (3b), an exhaust inlet (4a), an exhaust outlet (4b) and an end exhaust (4c), wherein the tubing system (2) is connected to the disinfection chamber (1a) via the ozone outlet (3b) and the exhaust inlet (4a), and is configured to, during an operation of the device by a user, supply to the disinfection chamber (1a) ozone gas via the ozone outlet (3b), and to receive from the disinfection chamber (1a) ozone gas via the exhaust inlet (4a);
   - an ozone generator (5) connected to the tubing system (2) and configured to generate ozone gas at a rate of between 1 grams of ozone per hour to 100 grams of ozone per hour, and preferably of between 1 grams of ozone per hour to 60 grams of ozone per hour, and supply via the ozone inlet (3a) said ozone gas to the tubing system (2);

   - an ozone destroy system (6) connected to the tubing system (2) and is configured to receive from the latter via the exhaust outlet (4b) ozone gas that has been received by the tubing system via the exhaust inlet (4a), and to convert said ozone gas to a non-toxic gaseous product, and to supply said non-toxic gaseous product to the end exhaust (4c);
   - sensing means (9) configured for sensing at least one operational parameter and generating and transmit a corresponding sensing signal;
   - a control unit (7) that comprises a power supply for powering the device, a computer (8a) and user command means (8b) configured for receiving operation commands from a user, the computer preferably being a programmable logic controller, and the control unit (7) is configured to control the operation of the device according to the operation commands, and is configured to receive the sensing signal and inhibit the operation of the system when the sensing signal does not correspond to a safety condition.
CLAUSE 2. A device according to clause 1, wherein the device is shaped and sized as a standalone closet.
CLAUSE 3. A device according to any of the preceding clauses, wherein the main body (1) is shaped as cuboid.
CLAUSE 4. A device according to any of the preceding clauses, wherein the device is shaped and sized as a benchtop instrument.
CLAUSE 5. A device according to any of the preceding clauses, wherein the device has a height (H) of between 80 cm and 200 cm, and preferably of between 130 cm and 170 cm, and more preferably of between 145 cm and 160 cm, and most preferably of between 150 cm and 155 cm.
CLAUSE 6. A device according to any of the preceding clauses, wherein the device has a length (L) of between 50 cm and 180 cm, and preferably of between 90 cm and 150 cm, and more preferably of between 110 cm and 145 cm, and most preferably of between 130 cm and 140 cm, and/or is equal to the length (L1) of the main body (1).
CLAUSE 7. A device according to any of the preceding clauses, wherein the device has a width (W) that is of between 50 cm and 150 cm, and preferably of between 70 cm and 130 cm, and more preferably of between 80 cm and 110 cm, and most preferably of between 90 cm and 100 cm, and/or is equal to the width (W1) of the main body (1).
CLAUSE 8. A device according to any of the preceding clauses, wherein the device has at least four legs (15) supporting on top of them the main body (1), wherein preferably each leg has a height of between 1 cm and 15 cm, and more preferably of between 4 cm and 10 cm.
CLAUSE 9. A device according to any of the preceding clauses, wherein the disinfection chamber has a height (H1a) that is of between 80 cm and 200 cm, and preferably of between 130 cm and 170 cm, and more preferably of between 140 cm and 150 cm, and most preferably of between 140 cm and 145 cm, and/or is equal to the height (H1) of the main body (1).
CLAUSE 10. A device according to any of the preceding clauses, wherein the disinfection chamber has a length (L1a) that is of between 50 cm and 180 cm, and preferably of between 90 cm and 150 cm, and more preferably of between 100 cm and 125 cm, and most preferably of between 100 cm and 110 cm.
CLAUSE 11. A device according to any of the preceding clauses, wherein the disinfection chamber (1a) has a width (W1a) that is of between 50 cm and 150 cm, and preferably of between 70 cm and 130 cm, or of between 60 cm and 70 cm, and more preferably of between 90 cm and 120 cm, and most preferably of between 100 cm and 110 cm. CLAUSE 12. A device according to any of the preceding clauses, wherein the main body (1) comprises a compartment (1e) that is adjacent to the disinfection chamber (1a) and contains within it any or both of the ozone generator (5) and the ozone destroy means (6).
CLAUSE 13. A device according to clause 12, wherein the compartment (1e) is separated from the disinfection chamber by a separation wall there between.
CLAUSE 14. A device according to any of clauses 12-13, wherein the compartment (1e) has a height (H1e) that is equal or smaller or larger than, to the height (H1a) of the disinfection chamber (1a).
CLAUSE 15. A device according to any of clauses 12-14, wherein the compartment (1e) has a length (L1e) that is equal to, or is smaller or larger than, the length (L1a) of the disinfection chamber (1a).
CLAUSE 16. A device according to any of clauses 12-15, wherein the compartment (1e) has a width (W1e) that is equal to, or smaller or larger than, the width (W1a) of the disinfection chamber (1a).
CLAUSE 17. A device according to any of clauses 12-16, wherein the main body comprises a service entrance (1f) configured to allow the user accessing the compartment (1e).
CLAUSE 18. A device according to any of clauses 12 -17, wherein the compartment (1e) is located behind the user command means (8).
CLAUSE 19. A device according to any of clauses 12-18, wherein the main body (1) and the compartment (1e) have an exhaust opening (1g) wherein the end exhaust (4c) is located.
CLAUSE 20. A device according to any of clauses 12-19, wherein at least part of the tubing system (2) extends from the disinfection chamber (1a) to within the compartment (1e).
CLAUSE 21. A device according to any of the clauses 12-20, wherein the disinfection chamber (1a) is located in front of the compartment (1e) along the width (W) of the main body (1).
CLAUSE 22. A device according to any of clauses 12-21, wherein the disinfection chamber (1a) and the compartment (1e) have in common two lateral and vertical walls which are located opposite to each other across the length (L1a) of the main body (1a).
CLAUSE 23. A device according any of clauses 12-22, wherein the main body (1) has in common with the disinfection chamber (1) and/or with the compartment (1e) a bottom floor and/or a top ceiling.
CLAUSE 24. A device according to any of the preceding clauses, wherein the door (1c) has a transparent window (1d) configured to allow the user seeing within the disinfection chamber (1a).
CLAUSE 25. A device according to any of the preceding clauses wherein the main body (1) has a control part (1h) that preferably extends fully across the width (W1a) and/or the height (H1a) of the main body (1), the user command means (8) being located in said control part (1h), and said control part (1h) being distinct from the disinfection chamber (1a) and/or the compartment (1e).
CLAUSE 26. A device according to clause 25, wherein:
   - the control part (1h) has a length (L1h) that is of between 20 cm and 40cm, and preferably of between 30 cm and 35 cm; and/or
   - the control means (8) and the door (1c) are both located on a side of the main body (1).
CLAUSE 27. A device according any of the preceding clauses, wherein the interior of the disinfection chamber (1a) where the wearables are to be inserted is made of a material that preferably comprises any of an oxidation resistive material, a stainless steel, a chromium-nickel stainless steel, stainless steel 316, stainless steel doped with molybdenum and combinations therefrom.
CLAUSE 28. A device according to any of the preceding clauses, wherein the interior of the disinfection chamber (1a) in which the wearables are to be inserted has a volume of between 300 liters (litres) and 2500 liters, and preferably of between 800 and 1200 liters, and most preferably of between 880 and 920 liters .
CLAUSE 29. A device according to any of the preceding clauses, wherein the disinfection chamber (1a) comprises within it a rack (22) configured for supporting on it wearables to be disinfected.
CLAUSE 30. A device according to any of the preceding clauses, wherein the disinfection chamber (1a) comprises in its interior a hanger (20) that preferably is a beam that is supported from two opposing walls of the disinfection chamber (1a) and extends lengthwise across the latter, and the hanger (20) is configured for hanging from the same wearables to be disinfected or bags or nets or other hangers or containers containing said wearables.
CLAUSE 31. A device according to clause 30, wherein the hanger comprises an S-formed hook that is configured for hanging from the same wearables to be disinfected or bags or nets or containers containing said wearables.
CLAUSE 32. A device according to any of the preceding clauses, wherein the disinfection chamber (1a) is hermetically sealed when the door (1c) is closed, thusly preventing the escape of ozone from within it via any route other than through the tubing system (2) connected to the disinfection chamber (1a), when the system is operated.
CLAUSE 33. A device according to any of the preceding clauses, wherein the main body (1) is hermetically sealed when the door (1c) is closed, thusly preventing the escape of ozone from within it, when the system is operated.
CLAUSE 34. A device according to any of the preceding clauses, wherein the ozone generator (5) comprises a precursor entrance (5a) via which a precursor gas enters the ozone generator (5) when the latter generates ozone, the precursor gas preferably comprising ambient air, and/or preferably wherein the ozone generator is preferably a corona discharge ozone generator.
CLAUSE 35. A device according to clause 34, wherein the precursor entrance communicates with a precursor opening at the disinfection chamber (1a), and via said precursor opening the precursor gas enters within the main body (1) and within the ozone generator (5).
CLAUSE 36. A device according to any of clauses 34-35, that further comprises an oxygen generator (10) that is configured to generate oxygen and supply said oxygen to the precursor entrance (5a), and said precursor gas comprises oxygen, the oxygen generator preferably comprising a zeolite molecular sieve (ZMS) configured to absorb nitrogen molecules from the air thusly producing high purity oxygen of preferred concentration of between 90% and 97%.
CLAUSE 37. A device according to clauses 36, wherein the control unit (7) is configured to control the oxygen generator (10).
CLAUSE 38. A device according to any of clauses 36-37, wherein the oxygen generator is configured to generate oxygen gas at rate of between 0.1 litre of oxygen per minute (L/min) and 100 L/min, and preferably of between 1 L/min and 10 L/min, and more preferably of between 4 L/min and 10 L/min, and most preferably the oxygen generator is configured to generate oxygen gas at rate of 10 L/min .
CLAUSE 39. A device according to any of the preceding clauses, wherein the power supply is configured to:
   - supply electrical power of between 1 kW and 50 kW, and preferably of between 5 kW and 15 kW, and most preferably 10 kW; and/or
   - operate under and an electrical supply to the same of AC voltage of a value of 380 V or of 220 V and/or of frequency of 50Hz.
CLAUSE 40. A device according to any of the preceding clauses, that comprises a 25 A circuit breaker.
CLAUSE 41. A device according any of the preceding clauses, that comprises a cooling system configured to cool the ozone generator when the latter and the system are operated, the cooling system preferably being a water cooling system or an air cooling system, and wherein the control unit and the cooling system are preferably configured so that the former controls the latter.
CLAUSE 42. A device according to any of the preceding clauses, that comprises an ultraviolet (UV) radiation source (21) located within the main body (1), and preferably on or within the disinfection chamber (1a), wherein the control unit (7) is configured to control the operation of the UV radiation source, and the latter when operated is configured to generate and supply UV radiation within the disinfection chamber (1a).
CLAUSE 43. A device according to clause 42, wherein the UV radiation comprises any of UV-A, UV-B and UV-C, and combinations thereof, and preferably UV-C.
CLAUSE 44. A device according to any of the preceding clauses, wherein the door (1c) comprises a UV filter configured to block UV-C and/or UV-B and/or UV-A radiation to pass through said door and/or any transparent window (1d) thereon, as for example according to clause 24, when the door is closed.
CLAUSE 45. A device according to any of the preceding clauses, that comprises a suction pump (12), that is connected to the tubing system (2) and is configured to be controllable by the control system (7) when the system is operated, the control system (7) being configured to control the suction pump (12), and wherein the suction pump (12) and the tubing system (2) are configured to do any of the following and combinations thereof when the system is operated:
   - force ozone gas and/or other gases, for example atmospheric air and/or oxygen, to move out of the disinfection chamber (1a) and through the exhaust inlet (4a) and through the exhaust outlet (4b) and towards and/or through the ozone destroy system (6);
   - evacuate, e.g. evacuate the gaseous atmosphere out of, the disinfection chamber (1a);
   - force atmospheric air and/or oxygen gas to move into and/or through the tubing system (2) or parts thereof and/or into the disinfection chamber (1a), preferably through the ozone outlet (3b);
   - force the non-toxic gaseous product to move out of the ozone destroy system (6) and/or out of the system and/or through the end exhaust (4c);
   - force atmospheric air and/or oxygen gas to move into and/or through the tubing system and out of the ozone destroy system (6);
   - force a precursor gas, such as for the example the precursor referred to in clause 34, to enter the ozone generator (5).
CLAUSE 46. A device according to any of the preceding clauses, wherein the sensing means (9) comprise a lock sensor configured to sense whether the door (1c) is fully closed or not, and the safety condition comprises or is that the door (1c) is fully closed.
CLAUSE 47. A device according to any of the preceding clauses, wherein the sensing means comprise an external ozone sensor that is located outside the disinfection chamber (1a) and preferably outside the main body (1), and is configured to measure the concentration of ozone thereat, and the safety condition comprises or is that the said concentration of ozone measured by the external ozone sensor is less than a safe external concentration that preferably is of 0.05 ppm (parts per million).
CLAUSE 48. A device according to any of the preceding clauses, wherein the sensing means comprise an internal ozone sensor that is located within the disinfection chamber (1a) or within the tubing system (2), and is configured to measure the concentration of ozone thereat, and preferably the safety condition is or comprises that the concentration of ozone measured by the internal ozone sensor is less than a maximum allowed internal concentration of preferably 10010 ppm or 4000 ppm or 3000 ppm or 2000 ppm.
CLAUSE 49. A device according to any of the preceding clauses wherein the sensing means are electronic and connected via wire to the control unit, and the sensing signal is an electrical signal transmitted via said wire, and the control unit and the sensing means are configured so that the control unit can detect and/or process said electric signal and/or can control the sensing means.
CLAUSE 50. A device according to any of the preceding clauses, wherein the ozone destroy system (6) comprises a catalytic ozone destructor (6b) comprising a catalyst wherein preferably the catalyst comprises manganese oxide and/or copper oxide. CLAUSE 51. A device according to any of the preceding clauses, wherein the ozone destroy system (6) comprises a deodorization system (6a), such as for example a deodorization filter, that is configured to deodorize or aromatize the non-toxic gaseous product.
CLAUSE 52. A device according to any of the preceding clauses, that comprises a ventilation system (23) configured to homogenize the ozone gas concentration within the disinfection chamber when the system and the ozone generator are operated.
CLAUSE 53. A device according to clause 52, wherein the ventilation system comprises an electric fan located in the disinfection chamber or in or connected to the tubing system, and is configured to increase the travel speed or flow of any gas, such as ozone, that enters via the ozone outlet or is within the disinfection chamber.
CLAUSE 54. A device according to any of the preceding clauses wherein the user command means (8b) comprise a power button (8ba) or knob and the like configured for switching on or off the power supply.
CLAUSE 55. A device according to any of the preceding clauses, wherein the user command means (8b) comprise an emergency button (8bb) configured for stopping all operation of the device without interrupting the power supply of the same.
CLAUSE 56. A device according to any of the preceding clauses, wherein the user command means (8b) comprise a reset button (8bc) configured for restarting operation of the system or of the control unit or of the computer therein in case of a failing, and preferably for security reasons said reset button has to be used by the user every time the device is switched on.
CLAUSE 57. A device according to any of the preceding clauses, wherein the user command means (8b) which preferably comprise an electronic touchscreen (8bd) are configured to display to the user several operation command options and sense a touching or selection of any of said operation command options by the user, and the computer is configured to convert said touching or selection of any of said operation command options to a corresponding operation command that is pre-stored and/or programmed in the computer.
CLAUSE 58. A device according to any of the preceding clauses, wherein the tubing system (2) comprises an air vent with an air vent valve (14) therein configured to let air enter the tubing system and/or the disinfection chamber when the air vent valve is open, wherein said air vent valve is controllable by the control unit or is mechanical.
CLAUSE 59. A device according to any of the preceding clauses, wherein said device further comprises a heating system that is configured to increase the temperature inside the disinfection chamber (1a).
CLAUSE 60. A device according to clause 60, wherein the heating system is connected to and configured to be controllable by the control unit (7a), and the latter is configured to control the heating system.
CLAUSE 61. A device according to any of clauses 59-60, wherein the heating system comprises a heating element installed (located) inside the disinfection chamber (1a) or inside the tubing system (2).
CLAUSE 62. A device according to clause 61, wherein the heating element comprises an electrical resistance that preferably is configured to operate at a nominal power in the range of 1kW to 10 kW, and more preferably in the range of 1 kW to 3 kW.
CLAUSE 63. A device according to any of clauses 59-62, wherein the heating system comprises a temperature sensor that is configured to measure the temperature inside the disinfection chamber (1a) or inside the tubing system (2).
CLAUSE 64. A device according to any of clauses 59-63, wherein the heating system comprise an auxiliary suction pump that is configured to force any gas from inside the disinfection chamber (1a) to flow by the heating element and to subsequently flow inside the disinfection chamber (1a).
CLAUSE 64. A device according to clauses 59-63, wherein the tubing system (2) comprises a tubing circuit, and wherein the heating element and the auxiliary pump are installed (located, connected to) at said tubing circuit, and the tubing system and heating system, when the latter is operated, are configured to extract (any) gas from the disinfection chamber via said auxiliary suction pump, pass said gas via the tubing circuit and the heating element thereat, heat said gas with the heating element, and reintroduce the thusly heated gas into the disinfection chamber.
CLAUSE 65. A device according to clause 64, wherein the tubing circuit comprises a tubing circuit inlet connected to the disinfection chamber, a tubing circuit outlet connected to the disinfection chamber, and the auxiliary suction pump when operated is configured to extract gas from the disinfection chamber via said tubing circuit inlet, and reintroduce the gas intro disinfection chamber via said tubing circuit outlet.
CLAUSE 66. A device according to any of clauses 59-65, wherein the temperature inside the disinfection chamber is the temperature of a gaseous atmosphere inside said disinfection chamber or inside the tubing circuit connected to said disinfection chamber, or is the temperature of any part of said gaseous atmosphere, and further preferably is the temperature measured by the temperature sensor described in clause 63. CLAUSE 67. A device according to any of clauses 59-66, wherein the heating system is configured to increase the temperature inside the disinfection chamber to a desired disinfection temperature, said desired disinfection temperature preferably being of between 25°C and 100°C, and more preferably of between 40°C to 90°C, and most preferably of between 60°C and 90°C.
CLAUSE 68. A device according to any of the preceding clauses and clause 57 and clause 58 and clause 41 and clause 45 and clause 48 and any of clauses 36-38 and any of clauses 52-53 and clause 67, wherein one of said pre-stored and/or programmed in the computer operation commands comprises instructions for any of the following or combinations thereof or preferably all of:
   - increasing the temperature inside the disinfection chamber to the desired disinfection temperature, using the heating system;
   - generating oxygen using the oxygen generator (10);
   - generating with the ozone generator ozone gas for an ozone generation duration of between 1 second and 10 minutes (min), or for a disinfection duration of between 1 minute (min) and 20 min, and preferably of between 2 min and 10 min, or for a total duration of between 1 second and 30 minutes, while measuring with the internal ozone sensor an ozone concentration of between 1 ppm and 10000 ppm;
   - while generating with the ozone generator ozone gas, operating the cooling system and using the same to cool the ozone generator;
   - while generating with the ozone generator ozone gas, using the ventilation system to homogenize the ozone gas concentration within the disinfection chamber;
   - after the disinfection duration or the ozone generation duration or the total duration has passed, disconnecting or shutting down or stop operating the ozone generator and/or the oxygen generator and/or the cooling system and/or the ventilation system, and operating the pump to remove ozone from the disinfection chamber, and open the air vent valve when the latter is controllable by a computer, and operating the ozone destroy system (6) thusly destroying ozone gas until the internal ozone sensor measures an ozone concentration of less than 0.05 ppm and/or until a safety period of time has passed;
   - when the internal ozone sensor measures an ozone concentration of less than 0.05 ppm, switch off the pump and preferably close the air vent valve when the latter is controllable by the computer;
   - indicate to the user via the touchscreen that the operation command has been executed.
CLAUSE 69. A device according to any of the preceding clauses, wherein the wearables are clothes.
CLAUSE 70. A device according to any of the preceding clauses, wherein the wearables are any of clothes, apparel, clothing accessories, trousers, shoes, hair caps, personal protective equipment (PPE) such as protective clothing, helmets, goggles, face masks or other garments or equipment designed to protect the wearer's body from injury or infection, and combinations thereof.
CLAUSE 71. A device according to any of the preceding clauses, that comprises any or both of:
   - a humidity sensor located within the disinfection chamber or attached to or within the tubing system and configured to measure there at the humidity concentration and generate and transmit a corresponding humidity signal;
   - a humidity control system configured to control the humidity within the disinfection chamber by introducing humidity, or removing humidity from, within the disinfection chamber;
      wherein preferably the control unit is configured to control the humidity sensor and receive said humidity signal, and/or is configured to control the operation of the humidity control system, the latter being configured to be controlled by the control unit, the control unit being preferably configured to operate the humidity control system to cause that the humidity concentration measured by the humidity sensor acquires a preferred humidity concentration value, the preferred humidity concentration value preferably being of between 8% and 80%, and more preferably of between 10% and 70%, and most preferably of between 10% and 40%.
CLAUSE 72. A system according to any of the preceding clauses, wherein the operation command includes specifying a type of item or wearable or characteristic therein such as a material of the wearable, and/or adjusting a recipe parameter according to said specifying item or wearable or characteristic, said recipe parameter being any of the following and combination thereof:
   - disinfection time;
   - ozone generation rate;
   - preferred Humidity value or range;
   - maximum allowed internal concentration of ozone (as measured by an internal ozone concentration sensor);
   - ozone concentration value or value range as measured by an internal ozone sensor;
   - ozone generation duration;
   - safety period of time.
   - Desired disinfection temperature.
   - Heating duration
CLAUSE 73. A system according to any of the preceding clauses wherein the operation command may comprise accessing a computer file according to which an initial condition, said condition being any of a specific material to be disinfected, or an item to be disinfected, or a volume or quantity of items to be disinfected, or a specific level of disinfection required, or a specific protocol of disinfection required, is correlated with at least one value or value range of any of the recipe parameters described in clause 72 or combinations thereof, and the operation command may further comprise controlling the system and components thereof as required, to realize, as part of implementing a disinfection cycle, the recipe parameter value or value range that correlates to the initial condition.
CLAUSE 74. A device (i.e. an apparatus or system) for disinfecting items, such as wearables, the device comprising:
   - a disinfection chamber (1a) which has a door (1c) and is configured for receiving within it, meaning to its interior, the items to be disinfected;
   - an ozone generator (5) connected to the disinfection chamber (1a) and configured to generate ozone gas and supply the latter to within (the interior of) the disinfection chamber (1a) when the system is operated;
   - an ozone destroy system (6) connected to disinfection chamber (1a) and configured to receive from within (the interior of) the latter ozone gas and to convert said ozone gas to a non-toxic gaseous product, and to preferably release said non-toxic gaseous product;
   - sensing means (6) configured for sensing at least one operational parameter and generating and transmit a corresponding sensing signal;
   - a control unit (7a) that comprises a power supply for powering the device, a computer and user command means (8) configured for receiving operation commands from a user, the computer preferably being a programmable logic controller, and the control unit is configured to control the operation of the device according to the operation commands, and is configured to receive the sensing signal and inhibit the operation of the system when the sensing signal does not correspond to a safety condition.
CLAUSE 75. A device according to clause 74, further comprising a heating system that when operated is configured for increasing the temperature inside the disinfection chamber, said heating system being configured to be controllable by the control unit 7a, said control unit being configured to control said heating system.
CLAUSE 76. A truck for disinfecting wearables, the truck comprising a device that is according to any of the preceding clauses and is loaded in/on a trunk (30) of said truck, the truck preferably being configured to supply sufficient power to the power supply unit of the device for powering and operating the latter.
CLAUSE 77. A method for disinfecting wearables, particularly personal protective equipment, used in a facility, the method comprising:
   - driving a truck that is according to clause 76 towards the facility that may be a healthcare establishment;
   - moving the wearables from the facility to the truck;
   - disinfecting the wearables using the truck and the device therein;
      and preferably,
   - moving the disinfected wearables from the truck to the facility.
CLAUSE 78. A method for disinfecting wearables, particularly personal protective equipment, used in a first and a second or additional facilities, the method comprising:
   - driving a truck that is according to clause 76 towards the first facility that may be a first healthcare establishment;
   - moving wearables from the first facility to the truck;
   - disinfecting the wearables using the truck and the device therein;
   - moving the disinfected wearables from the truck to the first facility;
   - driving the truck towards the second facility that may be a second healthcare establishment;
   - moving wearables from the second facility to the truck;
   - disinfecting the wearables using the truck and the device therein;
   - moving the disinfected wearables from the truck to the second facility.
CLAUSE 79. A method for disinfecting an item or items, the method comprising:
   - moving and placing the item (or items) to be disinfected to within a disinfection chamber of a system that optionally and preferably is a device or truck according to any of clauses 1-66 and/or comprises a disinfection chamber that has a door via which the items are being placed, an ozone generator, an ozone destroy system and a control unit;
   - closing the door;
   - generating ozone gas using the ozone generator and supplying said ozone gas to the disinfection chamber;
   - passing said ozone gas from the disinfection chamber and converting said ozone gas to a non-toxic gaseous product, and preferably releasing said non-toxic gaseous product;
   - opening the door and removing the items from the disinfection chamber.
CLAUSE 80. A method according to clause 79, wherein the system comprises a heating system, and the method further comprises:
   - increasing the temperature inside the disinfection chamber using the heating system.
CLAUSE 81. A method according to clause 80, wherein increasing the temperature comprises increasing the temperature to a desired disinfection temperature that preferably is of between 25°C and 100°C, and more preferably is of between 40°C and 90°C, and most preferably is of between 60°C and 90°C.
CLAUSE 82. A method according to any of clauses 79-81, wherein the items preferably are wearable, and more preferably are wearable personal protection equipment item such as for example face masks.
CLAUSE 83. A method according to any of clauses 79-82, wherein moving and placing the items to be disinfected comprises:
   - placing the items in a receptacle, wherein said receptacle preferably is a bag, and more preferably is a net bag, i.e. string bag;
   - moving said receptacle to within the chamber.
CLAUSE 84. A method according to clauses 83, wherein moving said receptacle comprises avoiding touching the receptacle and/or the contents of the receptacle.
CLAUSE 85. A method according to any of clauses 83-84, wherein removing the items comprises avoiding touching the contents of the receptacle.
CLAUSE 86. A method according to any of clauses 83-85, wherein removing the items may optionally comprise avoiding touching the receptacle.
CLAUSE 87. A method according to any of clauses 83-86, wherein the receptacle or bag preferably has a hook, such an S-shaped hook attached to it.
CLAUSE 88. A method according to any of clauses 83-87, wherein moving and placing the items to be disinfected may optionally further comprise any of the following:
   - attaching an S-hook to the receptacle;
   - holding the S-hook avoiding touching the receptacle and its contents; and optionally,
   - hanging said S-hook within the disinfection chamber.
CLAUSE 89. A method according to any of clauses 83-88, wherein removing the items from the disinfection chamber may comprise any of the following:
   - holding the S-hook avoiding touching the receptacle and/or its contents (the items/wearables) ;
   - un-hanging the S-hook from within the disinfection chamber.
CLAUSE 90. A method according to any of clauses 83-89, wherein, the disinfection chamber has at least one hanger configured so that the receptacle or an S-hook with said receptacle can be hanged from said hanger.
CLAUSE 91. A method according to any of clauses 79-90, wherein moving the item to be disinfected may optionally comprise any of the following and combinations thereof:
   - within a first facility such as for example a healthcare establishment such as for example a hospital, placing the items within a first package that for example is a first parcel or a sealable plastic bag;
   - transporting said first package to a second facility or vehicle that contains the disinfection chamber;
   - removing the items from the first package.
CLAUSE 92. A method according to any of clauses 79-91, wherein after removing the disinfected items from the chamber optionally the method comprises any of the following and combinations therefrom:
   - placing the items or a receptacle containing them within a second package that for example is a second parcel or a sealable plastic bag, while preferably avoiding touching the items or a receptacle containing them;
   - placing a sign on said second parcel indicating that the items are disinfected;
   - transporting said second package to the first facility.
CLAUSE 93. A method according to any of clauses 79-92, that further comprises wearing personal protective equipment while applying the method or parts thereof.
CLAUSE 94. A method according to any of clauses 79-93, wherein closing the door comprises locking the door.
CLAUSE 95. A method according to any of clauses 79-94, wherein generating ozone gas using the ozone generator comprises generating ozone gas at a rate of between 1 grams of ozone per hour to 100 grams of ozone per hour, and preferably of between 1 grams of ozone per hour to 60 grams of ozone per hour and supplying said ozone gas to the disinfection chamber.
CLAUSE 96. A method according to any of clauses 79-95, wherein generating ozone gas using the ozone generator and supplying said ozone gas to the disinfection chamber is optionally performed for a total duration of between 1 second and 20 minutes, or for an ozone generation duration of between 1 second and 10 minutes (min) or preferably of between 30 seconds and 5 min, or for a disinfection duration of between 1 second (s) and 20 min, and preferably of between 30 seconds and 10 min.
CLAUSE 97. A method according to any of clauses 79-96, wherein the method comprises supplying said ozone gas to the disinfection chamber at an ozone concentration of between 1 ppm and 10000 ppm as measured by an ozone sensor thereat, e.g. using an internal ozone sensor located at the disinfection chamber or at a tubing system that connects the ozone generator and the disinfection chamber and via which the ozone gas is being supplied.
CLAUSE 98. A method according to any of clauses 79-97, wherein the method comprises stopping passing said ozone gas from the disinfection chamber and converting said ozone gas to a non-toxic gaseous product until the zone concentration within the disinfection chamber as measured by said internal ozone sensor is less than 0.05 ppm, or until a safety period of time.
CLAUSE 99. A method according to any of clauses 79-98, wherein the method further comprises while supplying said ozone gas to the disinfection chamber controlling the humidity within the disinfection chamber by introducing humidity, or removing humidity from, within the disinfection chamber.
CLAUSE 100. A method according to clause 99 wherein controlling the humidity comprises causing that the humidity concentration within a chamber to acquire a preferred humidity concentration value which preferably is of between 8% and 80%, and more preferably of between 10% and 70%, and most preferably of between 10% and 40%.
CLAUSE 101. A method according to any of clauses 77-100, wherein the facility or the first facility or the second facility, is any of a healthcare facility, a hospital, a medical center, a public service building, a school, a factory, a store, a laundry shop, a goods storage facility, a food production or processing facility or open field, and the wearables are any of clothes, apparel, trousers, shoes, hair caps, personal protective equipment (PPE) such as protective clothing, helmets, goggles, face masks or other garments or equipment designed to protect the wearer's body from injury or infection, and combinations thereof.
CLAUSE 102. A system for sanitizing wearables, comprising:
   - a main body (1) that has a height (H1), a length (L1) and a width (W1), and comprises a disinfection chamber (1a) which has a door (1c) and is configured for receiving within it and via the door (1c) the wearables to be sanitized;
   - a tubing system (2) that has an ozone inlet (3a), an ozone outlet (3b), an exhaust inlet (4a), an exhaust outlet (4b) and an end exhaust (4c), wherein the tubing system (2) is connected to the disinfection chamber (1a) via the ozone outlet (3b) and the exhaust inlet (4a), and is configured to, during an operation of the system by a user, supply to the disinfection chamber (1a) ozone gas via the ozone outlet (3b), and to receive from the disinfection chamber (1a) ozone gas via the exhaust inlet (4a), the tubing system (2) further comprising a tubing circuit that has a circuit inlet and a circuit outlet, said tubing circuit being connected to the disinfection chamber (1a) via the circuit inlet and the circuit outlet;
   - a heating system that when operated is configured to increase a temperature inside the disinfection chamber (1a), the heating system comprising a heating element, an auxiliary suction pump, and a temperature sensor, the heating element when operated being configured to heat inside (the interior of, the inside of) the tubing circuit, the auxiliary suction pump when operated being configured to force any gas from inside the disinfection chamber (1a) to successively exit said disinfection chamber via said circuit inlet, flow inside said tubing circuit, and reenter the disinfection chamber via the circuit outlet;
   - an ozone generator (5) connected to the tubing system (2) and configured to generate ozone gas at an ozone generation rate of between 1 grams of ozone per hour to 100 grams of ozone per hour, and supply via the ozone inlet (3a) said ozone gas to the tubing system (2);
   - an ozone destroy system (6) that is connected to the tubing system (2) and is configured to receive from the latter via the exhaust outlet (4b) ozone gas that has been received by the tubing system via the exhaust inlet (4a), and to convert said ozone gas to a non-toxic gaseous product, and to supply said non-toxic gaseous product to the end exhaust (4c);
   - sensing means (9) configured for sensing at least one operational parameter and generating and transmit a corresponding sensing signal;
   - a control unit (7) that comprises a power supply for powering the system, a computer (8a) and user command means (8b) configured for receiving operation commands from a user, and the control unit (7) is configured to control the operation of the system according to the operation commands, and is configured to receive the sensing signal and inhibit the operation of the system when the sensing signal does not correspond to a safety condition.
103. A system according to clause 102, wherein the temperature inside the disinfection chamber is the temperature of a gaseous atmosphere inside said disinfection chamber or inside the tubing circuit connected to said disinfection chamber, and the temperature sensor when operated is configured to measure said temperature, and the heating system is configured to increase the temperature inside the disinfection chamber to a desired disinfection temperature of between 40°C to 100°C.
104. A system according to any of clauses 102-103, wherein the disinfection chamber (1a) comprises within it a rack (22) configured for supporting on it wearables to be disinfected, and the disinfection chamber (1a) further comprises in its interior a hanger (20) that is configured for hanging from the same wearables to be disinfected or bags or nets or other hangers or containers containing said wearables.
105. A system according to any of clauses 102-104, wherein the disinfection chamber (1a) is hermetically sealed when the door (1c) is closed, thusly preventing the escape of ozone from within it via any route other than through the tubing system (2) connected to the disinfection chamber (1a) when the system is operated
106. A system according to any of clauses 102-105, wherein the ozone generator (5) comprises a precursor entrance (5a) via which a precursor gas that comprises oxygen enters the ozone generator (5) when the latter generates ozone, and the system comprises an oxygen generator (10) that is configured to generate oxygen at rate of between 0.1 L/min and 100 L/min and to supply said oxygen to the precursor entrance (5a).
107. A system according to any of clauses 102-106, that comprises a water cooling system configured to cool the ozone generator when the latter and the system are operated, wherein the control unit and the cooling system are configured so that the former controls the latter.
108. A system according to any of clauses 102 - 107, that comprises a suction pump (12) that is connected to the tubing system (2) and is configured to be controllable by the control system (7) when the system is operated, the control system (7) being configured to control the suction pump (12), and wherein the suction pump (12) and the tubing system (2) are configured to, when the suction pump is operated, force ozone gas and/or any other gases to move out of the disinfection chamber (1a) and through the exhaust inlet (4a) and through the exhaust outlet (4b) and towards and through the ozone destroy system (6).
109. A system according to any of clauses 102-108, wherein the sensing means (9) comprise a lock sensor configured to sense whether the door (1c) is fully closed or not, and the safety condition comprises that the door (1c) is fully closed.
110. A system according to any of clauses 102-109, wherein the sensing means comprise an internal ozone sensor that is located within the disinfection chamber (1a) or within the tubing system (2) and is configured to measure the concentration of ozone thereat, and an external ozone sensor that is located outside the disinfection chamber (1a) and is configured to measure the concentration of ozone thereat, and the safety condition comprises that the concentration of ozone measured by the external ozone sensor is less than a safe external concentration of 0.05 ppm.
111. A system according to any of clauses 102-110, wherein the ozone destroy system (6) comprises a catalytic ozone destructor (6b) comprising a catalyst, and the system comprises a ventilation system (23) configured to homogenize the ozone gas concentration within the disinfection chamber when the system and the ozone generator are operated.
112. A system according to any of clauses 102 - 111, that comprises:
   - a humidity sensor located within the disinfection chamber or within the tubing system and configured to measure thereat a humidity concentration and generate and transmit a corresponding humidity signal;
   - a humidity control system configured to control the humidity within the disinfection chamber by introducing humidity, or removing humidity from, within the disinfection chamber;
      wherein the control unit is configured to control the humidity sensor and receive said humidity signal, and is configured to control the operation of the humidity control system, the latter being configured to be controlled by the control unit, the control unit being configured to operate the humidity control system to cause that the humidity concentration measured by the humidity sensor acquires a preferred humidity concentration value, the preferred humidity concentration value being of between 8% and 80%.
113. A system according to any of clauses 102-112, wherein the tubing system (2) comprises an air vent with an air vent valve (14) therein configured to let air enter the disinfection chamber when the air vent valve is open, wherein said air vent valve is controllable by the control unit or is mechanical
114. A system according to clauses 103, 106, 108, 110, and 113, wherein the user command means (8b) comprise an electronic touchscreen (8bd) and are configured to display to the user several operation command options and sense a selection of any of said operation command options by the user, and the computer is configured to convert said selection of any of said operation command options to a corresponding operation command that is pre-stored and/or programmed in the computer, wherein one of said operation commands comprises instructions for any of the following:
   - increasing the temperature inside the disinfection chamber to the desired disinfection temperature, using the heating system;
   - generating oxygen using the oxygen generator (10);
   - generating with the ozone generator ozone gas for a total duration of between 1 second and 20 minutes, while measuring with the internal ozone sensor an ozone concentration of between 1 ppm and 10000 ppm;
   - after the total duration has passed, stopping operating the ozone generator, and operating the suction pump to remove ozone from the disinfection chamber, and opening the air vent valve when the latter is controllable by the control unit, and operating the ozone destroy system (6) thusly destroying ozone gas until the internal ozone sensor measures an ozone concentration of less than 0.05 ppm and/or until a safety period of time has passed;
   - switching off the suction pump when the internal ozone sensor measures an ozone concentration of less than 0.05 ppm;
   - indicating to the user via the touchscreen that the operation command has been executed.
115. A system according to clause 114, wherein the operation command includes specifying a type of wearable or characteristic therein, and includes adjusting a recipe parameter according to said specifying wearable or characteristic, said recipe parameter being any of the following and combination thereof:
   - the ozone generation rate;
   - the preferred humidity concentration value;
   - the ozone concentration value measured by the internal ozone sensor;
   - the total duration;
   - the safety period of time;
   - the desired disinfection temperature;
   - a duration of operating the heating system,
   and wherein the operation command comprises accessing a computer file according to which an initial condition is correlated with at least one value or value range of any of said recipe parameters, and the operation command further comprises controlling the system and components thereof as required, to realize, as part of implementing a sanitization cycle, the recipe parameter value or value range that correlates to the initial condition, said initial condition being any of a specific material to be sanitized, a wearable to be sanitized, a volume or quantity of wearables to be sanitized, a specific level of sanitization required, or a specific protocol of sanitization required.
116. A system according to any of clauses clause 102-115, that comprises an ultraviolet (UV) radiation source (21) located within the main body (1), and preferably on or within the disinfection chamber (1a), wherein the control unit (7) is configured to control the operation of the UV radiation source, and the latter when operated is configured to generate and supply UV radiation within the disinfection chamber (1a).
117. A method for sanitizing a wearable, the method comprising:
   - moving and placing the wearable to be sanitized within a disinfection chamber of a system that is according to any of clauses 102-116 and comprises a disinfection chamber that has a door via which the items are being placed, a heating system, an ozone generator, an ozone destroy system and a control unit;
   - closing the door;
   - increasing the temperature inside the disinfection chamber using the heating system.
   - generating ozone gas using the ozone generator and supplying said ozone gas to the disinfection chamber;
   - passing said ozone gas from the disinfection chamber and converting said ozone gas to a non-toxic gaseous product, and releasing said non-toxic gaseous product;
   - opening the door and removing the items from the disinfection chamber.

The clauses belong to the specification (description) of the present application.

While the foregoing is directed to embodiments of the present invention, other and further embodiments of the invention may be devised without departing from the basic scope thereof.

## Claims

1. A system for sanitizing items, particularly wearables, comprising:
- a main body (1) that has a height (H1), a length (L1) and a width (W1), and comprises a disinfection chamber (1a) which has a door (1c) and is configured for receiving within it and via the door (1c) the items to be sanitized;
- a tubing system (2) that has an ozone inlet (3a), an ozone outlet (3b), an exhaust inlet (4a), an exhaust outlet (4b) and an end exhaust (4c), wherein the tubing system (2) is connected to the disinfection chamber (1a) via the ozone outlet (3b) and the exhaust inlet (4a), and is configured to, during an operation of the system by a user, supply to the disinfection chamber (1a) ozone gas via the ozone outlet (3b), and to receive from the disinfection chamber (1a) ozone gas via the exhaust inlet (4a), the tubing system (2) further comprising a tubing circuit that has a circuit inlet and a circuit outlet, said tubing circuit being connected to the disinfection chamber (1a) via the circuit inlet and the circuit outlet;
- a heating system that when operated is configured to increase a temperature inside the disinfection chamber (1a), the heating system comprising a heating element, an auxiliary suction pump, and a temperature sensor, the heating element when operated being configured to heat inside (the interior of, the inside of) the tubing circuit, the auxiliary suction pump when operated being configured to force any gas from inside the disinfection chamber (1a) to successively exit said disinfection chamber via said circuit inlet, flow inside said tubing circuit, and reenter the disinfection chamber via the circuit outlet;
- an ozone generator (5) connected to the tubing system (2) and configured to generate ozone gas at an ozone generation rate of between 1 grams of ozone per hour to 100 grams of ozone per hour, and supply via the ozone inlet (3a) said ozone gas to the tubing system (2);
- an ozone destroy system (6) that is connected to the tubing system (2) and is configured to receive from the latter via the exhaust outlet (4b) ozone gas that has been received by the tubing system via the exhaust inlet (4a), and to convert said ozone gas to a non-toxic gaseous product, and to supply said non-toxic gaseous product to the end exhaust (4c);
- sensing means (9) configured for sensing at least one operational parameter and generating and transmit a corresponding sensing signal;
- a control unit (7) that comprises a power supply for powering the system, a computer (8a) and user command means (8b) configured for receiving operation commands from a user, and the control unit (7) is configured to control the operation of the system according to the operation commands, and is configured to receive the sensing signal and inhibit the operation of the system when the sensing signal does not correspond to a safety condition;
- a suction pump (12) that is connected to the tubing system (2) and is configured to be controllable by the control system (7) when the system is operated, the control system (7) being configured to control the suction pump (12), and wherein the suction pump (12) and the tubing system (2) are configured to, when the suction pump is operated, force ozone gas and/or any other gases to move out of the disinfection chamber (1a) and through the exhaust inlet (4a) and through the exhaust outlet (4b) and towards and through the ozone destroy system (6),
wherein the tubing system (2) comprises an air vent with an air vent valve (14) therein configured to let air enter the disinfection chamber when the air vent valve is open, wherein said air vent valve is controllable by the control unit (7) or is mechanical.

2. A system according to claim 1, wherein the temperature inside the disinfection chamber is the temperature of a gaseous atmosphere inside said disinfection chamber or inside the tubing circuit connected to said disinfection chamber, and the temperature sensor when operated is configured to measure said temperature, and the heating system is configured to increase the temperature inside the disinfection chamber to a desired disinfection temperature of between 40°C to 100°C.

3. A system according to any of claims 1-2, wherein the disinfection chamber (1a) comprises within it a rack (22) configured for supporting on it items to be disinfected, and the disinfection chamber (1a) further comprises in its interior a hanger (20) that is configured for hanging from the same items to be disinfected or bags or nets or other hangers or containers containing said items.

4. A system according to any of claims 1-3, wherein the disinfection chamber (1a) is hermetically sealed when the door (1c) is closed, thusly preventing the escape of ozone from within it via any route other than through the tubing system (2) connected to the disinfection chamber (1a) when the system is operated

5. A system according to any of claims 1-4, wherein the ozone generator (5) comprises a precursor entrance (5a) via which a precursor gas that comprises oxygen enters the ozone generator (5) when the latter generates ozone, and the system comprises an oxygen generator (10) that is configured to generate oxygen at rate of between 0.1 L/min and 100 L/min and to supply said oxygen to the precursor entrance (5a).

6. A system according to any of claims 1-5, that comprises a water cooling system configured to cool the ozone generator when the latter and the system are operated, wherein the control unit and the cooling system are configured so that the former controls the latter.

7. A system according to any of claims 1-7, wherein the sensing means (9) comprise a lock sensor configured to sense whether the door (1c) is fully closed or not, and the safety condition comprises that the door (1c) is fully closed.

8. A system according to any of claims 1-7, wherein the sensing means comprise an internal ozone sensor that is located within the disinfection chamber (1a) or within the tubing system (2) and is configured to measure the concentration of ozone thereat, and an external ozone sensor that is located outside the disinfection chamber (1a) and is configured to measure the concentration of ozone thereat, and the safety condition comprises that the concentration of ozone measured by the external ozone sensor is less than a safe external concentration of 0.05 ppm.

9. A system according to any of claims 1-8, wherein the ozone destroy system (6) comprises a catalytic ozone destructor (6b) comprising a catalyst, and the system comprises a ventilation system (23) configured to homogenize the ozone gas concentration within the disinfection chamber when the system and the ozone generator are operated.

10. A system according to any of claims 1 - 9, that comprises:
- a humidity sensor located within the disinfection chamber or within the tubing system and configured to measure thereat a humidity concentration and generate and transmit a corresponding humidity signal;
- a humidity control system configured to control the humidity within the disinfection chamber by introducing humidity, or removing humidity from, within the disinfection chamber;
wherein the control unit is configured to control the humidity sensor and receive said humidity signal, and is configured to control the operation of the humidity control system, the latter being configured to be controlled by the control unit, the control unit being configured to operate the humidity control system to cause that the humidity concentration measured by the humidity sensor acquires a preferred humidity concentration value, the preferred humidity concentration value being of between 8% and 80%.

11. A system according to claims 2, 5, and 8, wherein the user command means (8b) comprise an electronic touchscreen (8bd) and are configured to display to the user several operation command options and sense a selection of any of said operation command options by the user, and the computer is configured to convert said selection of any of said operation command options to a corresponding operation command that is pre-stored and/or programmed in the computer, wherein one of said operation commands comprises instructions for any of the following:
- increasing the temperature inside the disinfection chamber to the desired disinfection temperature, using the heating system;
- generating oxygen using the oxygen generator (10);
- generating with the ozone generator ozone gas for a total duration of between 1 second and 20 minutes, while measuring with the internal ozone sensor an ozone concentration of between 1 ppm and 10000 ppm;
- after the total duration has passed, stopping operating the ozone generator, and operating the suction pump to remove ozone from the disinfection chamber, and opening the air vent valve when the latter is controllable by the control unit, and operating the ozone destroy system (6) thusly destroying ozone gas until the internal ozone sensor measures an ozone concentration of less than 0.05 ppm and/or until a safety period of time has passed;
- switching off the suction pump when the internal ozone sensor measures an ozone concentration of less than 0.05 ppm;
- indicating to the user via the touchscreen that the operation command has been executed.

12. A system according to claim 11, wherein the operation command includes specifying a type of item or characteristic therein, and includes adjusting a recipe parameter according to said specifying item or characteristic, said recipe parameter being any of the following and combination thereof:
• the ozone generation rate;
• the ozone concentration value measured by the internal ozone sensor;
• the total duration;
• the safety period of time;
• the desired disinfection temperature;
• a duration of operating the heating system,
and wherein the operation command comprises accessing a computer file according to which an initial condition is correlated with at least one value or value range of any of said recipe parameters, and the operation command further comprises controlling the system and components thereof as required, to realize, as part of implementing a sanitization cycle, the recipe parameter value or value range that correlates to the initial condition, said initial condition being any of a specific material to be sanitized, an item to be sanitized, a volume or quantity of items to be sanitized, a specific level of sanitization required, or a specific protocol of sanitization required.

13. A system according to any of claims 1-12, that comprises an ultraviolet (UV) radiation source (21) located within the main body (1), and preferably on or within the disinfection chamber (1a), wherein the control unit (7) is configured to control the operation of the UV radiation source, and the latter when operated is configured to generate and supply UV radiation within the disinfection chamber (1a).

14. A method for sanitizing items, particularly wearables, the method comprising:
- moving and placing the items to be sanitized within a disinfection chamber of a system that is according to any of claims 1-13 and comprises a disinfection chamber that has a door via which the items are being placed, a heating system, an ozone generator, an ozone destroy system and a control unit;
- closing the door;
- increasing the temperature inside the disinfection chamber using the heating system.
- generating ozone gas using the ozone generator and supplying said ozone gas to the disinfection chamber;
- passing said ozone gas from the disinfection chamber to the ozone destroy system and converting said ozone gas to a non-toxic gaseous product, and releasing said non-toxic gaseous product;
- opening the door and removing the items from the disinfection chamber.

15. A method according to claim 14, wherein the items are any of clothes, apparel, clothing accessories, trousers, shoes, hair caps, personal protective equipment, protective clothing, helmets, goggles, face masks and combinations thereof.
